(19) Europäisches Patentamt
European Patent Office
Office européen des brevets

(11) EP 4 644 371 A1

# (12) EUROPEAN PATENT APPLICATION
published in accordance with Art. 153(4) EPC

(43) Date of publication:
**05.11.2025 Bulletin 2025/45**

(21) Application number: **23912222.9**

(22) Date of filing: **27.12.2023**

(51) International Patent Classification (IPC):
***C07D 211/58*** (2006.01) ***A61K 9/51*** (2006.01)
***A61K 9/127*** (2025.01) ***A61K 31/7088*** (2006.01)
***A61K 47/18*** (2017.01) ***A61K 47/34*** (2017.01)
***A61K 47/44*** (2017.01) ***A61K 48/00*** (2006.01)
***A61P 25/00*** (2006.01) ***A61P 25/08*** (2006.01)
***A61P 25/28*** (2006.01) ***A61P 43/00*** (2006.01)
***C12N 15/12*** (2006.01) ***C12N 15/88*** (2006.01)

(52) Cooperative Patent Classification (CPC):
**A61K 9/127; A61K 9/51; A61K 31/7088; A61K 47/18; A61K 47/34; A61K 47/44; A61K 48/00; A61P 25/00; A61P 25/08; A61P 25/28; A61P 43/00; C07D 211/58; C07K 14/435; C12N 15/88**

(86) International application number:
**PCT/JP2023/046859**

(87) International publication number:
**WO 2024/143444 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 JP 2022211994**
**15.03.2023 JP 2023041432**

(71) Applicant: **Astellas Pharma Inc.**
**Tokyo 103-8411 (JP)**

(72) Inventors:
- **FUMIYAMA, Hitoshi**
**Tokyo 103-8411 (JP)**
- **SAITO, Tomoyuki**
**Tokyo 103-8411 (JP)**
- **IMANISHI, Masashi**
**Tokyo 103-8411 (JP)**
- **INOUE, Makoto**
**Tokyo 103-8411 (JP)**
- **NOTE, Miyu**
**Tokyo 103-8411 (JP)**
- **MORIOKA, Shunsuke**
**Tokyo 103-8411 (JP)**
- **ENDO, Taisuke**
**Tokyo 103-8411 (JP)**
- **KATO, Tetsuro**
**Tokyo 103-8411 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) 4-AMINOPIPERIDINE COMPOUND, LIPID NANOPARTICLES THEREOF, AND PHARMACEUTICAL COMPOSITION CONTAINING SAME

(57) The present invention addresses the problem of providing: a compound that is a 4-aminopiperidine lipid; lipid nanoparticles containing the lipid; and a pharmaceutical composition useful for nucleic acid medicines and the like. The present inventors have discovered a compound that is a 4-aminopiperidine lipid or a salt thereof, examined lipid nanoparticles that have the potential of being formed into various pharmaceutical compositions, and revealed that the lipid that is the compound according to the present invention or a salt thereof can form lipid nanoparticles, and moreover, lipid nanoparticles in which nucleic acid is encapsulated (i.e., nucleic



Processed by Luminess, 75001 PARIS (FR)

(Cont. next page)

acid lipid nanoparticles). In addition, the nucleic acid lipid nanoparticles containing the lipid according to the present invention are expected to serve as a component of pharmaceutical compositions useful for prevention and/or treatment of astrocyte-related diseases.

## Description

Technical Field

The present invention provides a cationic lipid which is useful as a component of lipid nanoparticles (also called a compound or a salt thereof below), lipid nanoparticles thereof, lipid nanoparticles encapsulating a nucleic acid therein (also called nucleic acid lipid nanoparticles below), lipid nanoparticles encapsulating a nucleic acid which is useful for prevention and/or treatment and a pharmaceutical composition containing nucleic acid lipid nanoparticles.

Background Art

Nucleic acids are recently used or are about to be used for preventing and/or treating diseases. For example, because proteins are synthesized in the body based on messenger RNA (mRNA), mRNA has drawn attention as a pharmaceutical product which is used for the purpose of expressing a desired protein in target cells. It is, however, difficult to deliver a nucleic acid itself such as mRNA alone into cells, and thus lipid nanoparticles (LNPs) are used as a delivery technique for delivering a nucleic acid, especially mRNA, into target cells. As examples of application of nucleic acid lipid nanoparticles, COVID-19 vaccines and cancer vaccines have been put on the market or clinically developed. In the nucleic acid lipid nanoparticles, various lipids are used as a cationic lipid, which is one of the components (PTLs 1 to 3).

PTLs 1 and 2 disclose that the compound of the formula (I) or a salt thereof can be used as a cationic lipid for lipid nanoparticles. In Examples, the compound below is described.

[Chem. 1]

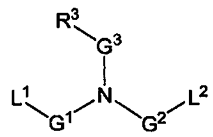

(I)

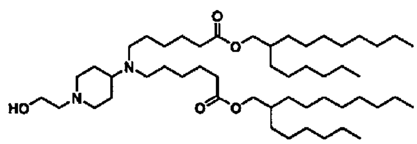

(Refer to the PTLs for the symbols in the formula.)

PTL 3 discloses that the compounds of the formulae below or a salt thereof can be used as a cationic lipid for lipid nanoparticles.

[Chem. 2]

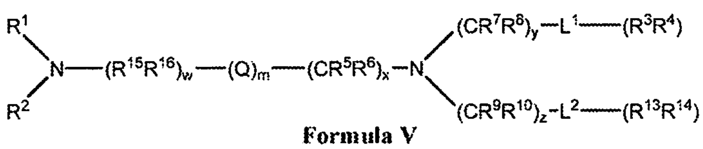

**Formula V**

(Refer to the PTL for the symbols in the formula.)

Astrocytes are one type of cells which are the most abundant in the central nervous system, play an important role in the homeostasis of blood-brain barrier and synapse formation of neurons in normal environment and have an important role of supporting the normal brain function (Frontiers in Cellular Neuroscience, 2022, vol. 16, 850866, Toxicologic Pathology, 2011, vol. 39(1), 115-123).

In the case of brain damage, however, astrocytes become activated, accumulate in the damaged site and form gliosis. Gliosis is believed to be a factor inhibiting neuronal regeneration and has been reported to be found in a wide range of central nervous system diseases such as spinal cord injury, neurodegenerative diseases including Alzheimer's disease and epilepsy (Neuron, 2014, vol. 81, P229-248). Accordingly, when an effective drug which is introduced into astrocytes and acts can be developed, a new therapeutic method for a wide range of central nervous system diseases may be provided.

Citation List

Patent Literature

[PTL 1] WO2021/204175A
[PTL 2] WO2021/204179A
[PTL 3] WO2022/081750A

Summary of Invention

Technical Problem

A major object of the invention is to provide a new cationic lipid which can be a component of lipid nanoparticles and create lipid nanoparticles for delivering a nucleic acid into cells. Another object of the invention is to provide nucleic acid lipid nanoparticles encapsulating a nucleic acid (for example, mRNA) and a pharmaceutical composition containing the same.

Another object of the invention is, for example, to provide a pharmaceutical composition containing lipid nanoparticles encapsulating a nucleic acid which is useful for preventing and/or treating an astrocyte-related disease (for example, mRNA).

Solution to Problem

As a result of intensive examination, the present inventors have found the compound of the formula (I) of the invention or a salt thereof and found that novel and useful lipid nanoparticles can be produced using the same as a cationic lipid. Moreover, the inventors have examined the proportions of the components of lipid nanoparticles (a cationic lipid, a neutral lipid, a PEGylated lipid and the like) and thus found an appropriate composition ratio. The invention can provide such a cationic lipid, lipid nanoparticles, nucleic acid lipid nanoparticles, a pharmaceutical composition containing nucleic acid lipid nanoparticles and the like.

That is, the invention relates to [1] to [21] below.

[1] A compound of a formula (I) or a salt thereof:

[Chem. 3]

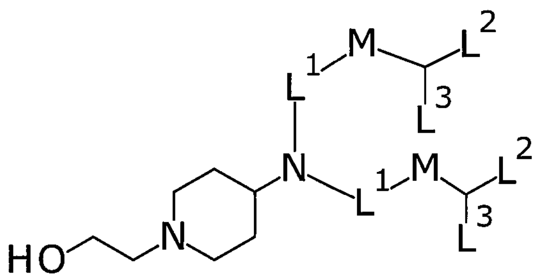

(I)

(in the formula,

$L^1$ is a $C_{5-10}$ alkylene,
$L^2$ and $L^3$ are each independently a $C_{5-15}$ alkyl, and
M is -C(=O)O- or -OC(=O)-).

[2] The compound or the salt thereof described in [1], in which $L^1$ in the formula (I) is a $C_{6-9}$ alkylene.
[3] The compound or the salt thereof described in [1] or [2], in which $L^2$ and $L^3$ in the formula (I) are each independently a $C_{6-10}$ alkyl.
[4] The compound or the salt thereof described in any of [1] to [3], in which the compound is selected from the group consisting of

di(heptadecan-9-yl) 8,8'- {[1-(2-hydroxyethyl)piperidin-4-y1]azanediyl } di(octanoate),

di(tridecan-7-yl) 9,9'- ([1-(2-hydroxyethyl)piperidin-4-yl]azanediyl}di(nonanoate) and {[1-(2-hydroxyethyl)piperidin-4-yl]azanediyl}di(nonane-9,1-diyl) bis(2-hexyldecanoate).

[5] The compound or the salt thereof described in [4], in which the compound is di(heptadecan-9-yl) 8,8'-{[1-(2-hydroxyethyl)piperidin-4-yl]azanediyl}di(octanoate).
[6] The compound or the salt thereof described in [4], in which the compound is di(tridecan-7-yl) 9,9'-{[1-(2-hydroxyethyl)piperidin-4-yl]azanediyl}di(nonanoate).
[7] The compound or the salt thereof described in [4], in which the compound is {[1-(2-hydroxyethyl)piperidin-4-yl] azanediyl}di(nonane-9,1-diyl) bis(2-hexyldecanoate).
[8] Lipid nanoparticles containing the compound or the salt thereof described in any of [1] to [7].
[9] The lipid nanoparticles described in [8] which further contain a neutral lipid and a PEGylated lipid.
[9-1] Lipid nanoparticles containing the compound or the salt thereof described in any of [1] to [7], a neutral lipid and a PEGylated lipid.
[10] The lipid nanoparticles described in [9] or [9-1] which encapsulate a nucleic acid.
[11] The lipid nanoparticles described in [10], in which the nucleic acid is mRNA.
[12] The lipid nanoparticles described in [10] to [11] which can express a protein in an astrocyte.
[13] The lipid nanoparticles described in [10], in which the nucleic acid is a nucleic acid which is useful for preventing and/or treating an astrocyte-related disease.
[13-1] The lipid nanoparticles described in [9-1] which encapsulate a nucleic acid which is useful for preventing and/or treating an astrocyte-related disease.
[14] Lipid nanoparticles encapsulating a nucleic acid and containing a compound of a formula (I) or a salt thereof, a neutral lipid and a PEGylated lipid,

wherein the nucleic acid is mRNA encoding NeuroD1 protein,
the compound of the formula (I) or the salt thereof is a compound selected from the group consisting of di(heptadecan-9-yl) 8,8'-{[1-(2-hydroxyethyl)piperidin-4-yl]azanediyl}di(octanoate), di(tridecan-7-yl) 9,9'-{[1-(2-hydroxyethyl)piperidin-4-yl]azanediyl } di(nonanoate) and {[1-(2-hydroxyethyl)piperidin-4-yl]azanediyl}di(nonane-9,1-diyl) bis(2-hexyldecanoate) or a salt thereof,
the neutral lipid is DSPC and cholesterol, and
the PEGylated lipid is DMG-PEG2000.

[15] Lipid nanoparticles according to [14],
where the compound of the formula (I) or the salt thereof is di(heptadecan-9-yl) 8,8'-{[1-(2-hydroxyethyl)piperidin-4-yl] azanediyl}di(octanoate) or a salt thereof,
[16] The lipid nanoparticles described in [11] or [14],
in which the nucleic acid is mRNA encoding NeuroD1 protein containing a base sequence encoding a protein having the amino acid sequence of SEQ ID NO: 2.
[17] The lipid nanoparticles described in [9] to [16] which contain 20.0 to 70.0 mol% of the compound or the salt thereof described in any of [1] to [7], 27.0 to 79.5 mol% of the neutral lipid and 0.5 to 3.0 mol% of the PEGylated lipid based on the total amount of the lipid nanoparticles.
[18] The lipid nanoparticles described in [9] to [16] which contain 35.0 to 70.0 mol% of the compound or the salt thereof described in any of [1] to [7], 27.5 to 64.0 mol% of the neutral lipid and 1.0 to 2.5 mol% of the PEGylated lipid based on the total amount of the lipid nanoparticles.
[19] A pharmaceutical composition containing the lipid nanoparticles described in any of [8] to [18].
[20] A pharmaceutical composition containing the lipid nanoparticles described in [8] to [18] and one or more pharmaceutically acceptable pharmaceutical additives.
[21] The pharmaceutical composition described in [19] or [20] for the prevention and/or the treatment of an astrocyte-related disease.
[22] A pharmaceutical composition containing the lipid nanoparticles described in [14] or [15] and one or more pharmaceutically acceptable pharmaceutical additives.

The invention also relates to
a pharmaceutical composition for preventing and/or treating an astrocyte-related disease which contains the lipid nanoparticles described in [8] to [18] containing the compound of the formula (I) or the salt thereof.
The pharmaceutical composition includes an agent for preventing and/or treating an astrocyte-related disease which contains the lipid nanoparticles described in [8] to [18] containing the compound of the formula (I) or the salt thereof.
The invention also relates to

use of the lipid nanoparticles described in [8] to [18] containing the compound of the formula (I) or the salt thereof for the manufacture of a pharmaceutical composition for preventing and/or treating an astrocyte-related disease,
the lipid nanoparticles described in [8] to [18] containing the compound of the formula (I) or the salt thereof for use in prevention and/or treatment of an astrocyte-related disease,
use of the lipid nanoparticles described in [8] to [18] containing the compound of the formula (I) or the salt thereof for the prevention and/or the treatment of an astrocyte-related disease and
a method for preventing and/or treating an astrocyte-related disease including administering an effective amount of the lipid nanoparticles described in [8] to [18] containing the compound of the formula (I) or the salt thereof to a subject.

Moreover, the invention relates to a method for delivering a nucleic acid (for example, a nucleic acid which is useful for preventing and/or treating an astrocyte-related disease) into cells of the body, in particular astrocytes, using nucleic acid lipid nanoparticles containing the compound of the formula (I) or a salt thereof.
The "astrocyte-related disease" is cerebral infarction, cerebral hemorrhage, traumatic brain injury, spinal cord injury, a neurodegenerative disease, epilepsy, amyotrophic lateral sclerosis, Duchenne muscular dystrophy, Alexander disease, multiple sclerosis or neuromyelitis optica. The astrocyte-related disease is, in an aspect, cerebral hemorrhage, traumatic brain injury, spinal cord injury, a neurodegenerative disease, epilepsy, amyotrophic lateral sclerosis, Duchenne muscular dystrophy, Alexander disease, multiple sclerosis or neuromyelitis optica.
Here, cerebral infarction is perforator infarction or cerebral infarction having brain damage in the penetrating branch territory in an aspect, subacute-phase cerebral infarction in an aspect, subacute-phase to chronic-phase cerebral infarction in an aspect, chronic-phase cerebral infarction in an aspect, cerebral infarction having motor dysfunction with high severity in an aspect, cerebral infarction with modified Rankin Scale (mRS) of 2 or more, or cerebral infarction with mRS of 4 or more in an aspect, cerebral infarction of a combination thereof in an aspect or perforator infarction, cerebral infarction having brain damage in the penetrating branch territory, subacute-phase to chronic-phase cerebral infarction or cerebral infarction having motor dysfunction with high severity in an aspect. The "acute stage" is a period having the possibility of worsening symptoms from immediately after the onset of cerebral infarction to less than 72 hours. The "subacute-phase" is a period from 72 hours after the onset of cerebral infarction before one month thereafter. The "subacute-phase to chronic-phase" is at and after 72 hours after the onset of cerebral infarction. The "chronic-phase" is the period at and after one month after the onset of cerebral infarction.
The invention is not limited to the aspects above and also includes aspects of appropriate combinations of the contents described in the detailed invention of the specification.
This specification includes the contents disclosed in Japanese patent application No. 2022-211994 and Japanese patent application No. 2023-041432, which the present application claims priorities from.

Advantageous Effects of Invention

The lipid which is the compound of the formula (I) or a salt thereof can form lipid nanoparticles as a component of the lipid nanoparticles, and the formed lipid nanoparticles can deliver a nucleic acid to target cells, such as astrocytes.
Using such lipid nanoparticles, a nucleic acid (for example, a nucleic acid which is useful for preventing and/or treating an astrocyte-related disease) can be delivered into target cells, and as a result, a pharmaceutical composition which is useful for preventing or treating an astrocyte-related disease can be provided. That is, the pharmaceutical composition containing the nucleic acid lipid nanoparticles of the invention can be used as an agent for preventing and/or treating an astrocyte-related disease.

Brief Description of Drawings

[Fig. 1] Fig. 1 shows the results of modified Rankin Scale (mRS) evaluation of daily change in motor dysfunction after endothelin-1 treatment (induction of cerebral ischemia) in a NeuroD1 (ND1) nucleic acid lipid nanoparticle group (ND1 mRNA) and a control group (Control mRNA) in Example 5. The horizontal axis shows the period after the endothelin-1 infusion (days), and the vertical axis shows the mRS values (maximum value of 6). The white circle shows the average value of mRS at each time point of evaluation of the ND1 nucleic acid lipid nanoparticle group. Here, the mRS was evaluated every two weeks on day 28 or later afterendothelin-1 infusion, but the results on day 56, indicates an average value of results of evaluating individuals on day 57, 58 or 59. The error bar represents standard error of the mean (SEM). The black circle shows mRS at each time point of evaluation of one individual of the control group. The results on day 56 indicates the result of performing evaluation on day 58. The nucleic acid lipid nanoparticles were administered on day 21 after the endothelin-1 infusion.

Description of Embodiments

The invention will be explained in detail below.

In this specification, the terms below have the meanings below unless otherwise specifically noted. The definitions below are for clarifying the defined terms but do not limit the terms. When the terms used here are not specifically defined, the terms are used with the meanings which are generally accepted by one skilled in the art. Unless otherwise specifically noted, when a symbol in a chemical formula in this specification is also used in another chemical formula, the same symbol has the same meaning.

The "alkyl" is a linear or branched alkyl. The $C_{5-10}$ alkyl is an alkyl having 5 to 10 carbon atoms. In an aspect, the alkyl is a $C_{5-10}$ alkyl. In an aspect, the alkyl is a $C_{5-15}$ alkyl. In an aspect, the alkyl is a $C_{6-10}$ alkyl. In an aspect, the alkyl is a $C_{6-8}$ alkyl. In an aspect, the alkyl is n-hexyl, n-octyl or n-decyl. In an aspect, the alkyl is n-hexyl or n-octyl.

The "alkylene" is a linear or branched alkylene. The $C_{5-10}$ alkylene is an alkylene having 5 to 10 carbon atoms, and the $C_{1-6}$ alkylene is an alkylene having 1 to 6 carbon atoms. In an aspect, the alkylene is a $C_{5-10}$ alkylene. In an aspect, the alkylene is a $C_{6-9}$ alkylene. In an aspect, the alkylene is a $C_{7-9}$ alkylene. In an aspect, the alkylene is a $C_{6-8}$ alkylene. In an aspect, the alkylene is hexanediyl, heptanediyl, octanediyl or nonanediyl. In an aspect, the alkylene is heptanediyl, octanediyl or nonanediyl. In an aspect, the alkylene is heptane-1,7-diyl, octane-1,8-diyl or nonane-1,9-diyl.

The "halogen" is F, Cl, Br or I.

The "lipid nanoparticles" are nanoparticles containing a lipid as a main component. In general, the lipid nanoparticles contain a cationic lipid, a neutral lipid and a PEGylated lipid. In an aspect, the lipid nanoparticles are nucleic acid lipid nanoparticles further encapsulating a nucleic acid. The "nucleic acid lipid nanoparticles" are lipid nanoparticles encapsulating a nucleic acid which is useful for preventing and/or treating a disease, lipid nanoparticles encapsulating a nucleic acid which is useful for preventing and/or treating an astrocyte-related disease in an aspect or lipid nanoparticles encapsulating mRNA in an aspect.

The "particle size" is the particle size of the lipid nanoparticles. The particle size is measured using a dispersion in still state with a particle size analyzer (Zetasizer Nano ZSP, manufactured by Malvern Panalytical) using the method of Dynamic Light Scattering (DLS) as a hydrodynamic diameter (Dh). The particle size is in accordance with ISO22412. The "particle size" of the lipid nanoparticles is calculated as a Z-average particle size. The particle size of the lipid nanoparticles is 10 nm to 1000 nm in an aspect, 30 nm to 500 nm in an aspect or 30 nm to 250 nm in an aspect. The particle size is 80 nm to 110 nm in an aspect. The particle size is 60 nm to 180 nm in an aspect. The particle size is 70 nm to 110 nm in an aspect.

The "cationic lipid" is a compound or a salt thereof which can carry a cation (positive electric charge) in the molecule in response to the pH and which has a linear or branched fatty acid chain or a fatty acid ester. In an aspect, the cationic lipid is a compound or a salt thereof which has an $\alpha$-branched structure at the side chain moiety ($L^2$ and $L^3$ side). In an aspect, the cationic lipid is the compound of the formula (I) or a salt thereof. Here, the $\alpha$-branched structure indicates that a hydrocarbon group is branched at the first carbon atom of the carbon atoms of the side chain side ($L^2$ and $L^3$ side) bonded to M, and the $\beta$-branched structure indicates that a hydrocarbon group is branched at the second carbon atom from the carbon atom at the side chain side ($L^2$ and $L^3$ side) of the carbon atoms bonded to M.

"Neutral lipids" are "phospholipids" and "sterols". In an aspect, the neutral lipid is a phospholipid and a sterol. In an aspect, the neutral lipid is a phospholipid, and in an aspect, the neutral lipid is a sterol.

The "phospholipid" is a lipid having a phosphoric acid ester group. Examples thereof include 1,2-distearoyl-sn-glycero-3-phosphocholine (DSPC), 1,2-dilinoleoyl-sn-glycero-3-phosphocholine, 1,2-dimyristoyl-sn-glycero-phosphocholine (DMPC), 1,2-dioleoyl-sn-glycero-3-phosphocholine (DOPC), 1,2-dierucoyl-sn-glycero-3-phosphocholine (DEPC), 1,2-dipalmitoyl-sn-glycero-3-phosphocholine (DPPC), 1,2-diundecanoyl-sn-glycero-phosphocholine (DUPC), 1-palmitoyl-2-oleoyl-sn-glycero-3-phosphocholine (POPC), 1-stearoyl-2-oleoyl-sn-glycero-3-phosphocholine (SOPC), 1,2-dioleoyl-sn-glycero-3-phospho-rac-(1-glycerol) sodium salt (DOPG), 1,2-dimyristoyl-sn-glycero-3-phospho-rac-(1-glycerol) sodium salt (DMPG), 1,2-dipalmitoyl-sn-glycero-3-phospho-rac-(1-glycerol) sodium salt (DPPG), 1,2-distearoyl-sn-glycero-3-phospho-rac-(1-glycerol) sodium salt (DSPG), 1,2-dioleoyl-sn-glycero-3-phosphoethanolamine (DOPE), 1,2-dimyristoyl-sn-glycero-3-phosphoethanolamine (DMPE), 1,2-dipalmitoyl-sn-glycero-3-phosphoethanolamine (DPPE), 1,2-distearoyl-sn-glycero-3-phosphoethanolamine (DSPE), 1-palmitoyl-2-oleoyl-sn-glycero-3-phosphoethanolamine (POPE), 1,2-dierucoyl-sn-glycero-3-phosphoethanolamine (DEPE), 1,2-dioleoyl-sn-glycero-3-phospho-L-serine (DOPS) and sphingomyelin or the like. In an aspect, the phospholipid is DSPC. In an aspect, the phospholipid is DOPE.

The "sterol" is a steroid alcohol and is a compound having a hydroxy group on the A ring of a steroid skeleton. In an aspect, the sterol is cholesterol and a corticosteroid. In an aspect, the sterol is fecosterol, sitosterol, ergosterol, campesterol, stigmasterol, brassicasterol, tomatidine, tomatin, ursolic acid, $\alpha$-tocopherol, a mixture thereof or the like. Moreover, the sterol is a combination of two or more kinds. In an aspect, the sterol is cholesterol.

The "PEGylated lipid" refers to a lipid having polyethylene glycol (PEG). The PEGylated lipid is a lipid modified with polyethylene glycol. The PEGylated lipid is PEG-modified phosphatidylethanolamine, PEG-modified phosphatidic acid, PEG-modified ceramide, PEG-modified dialkylamine, PEG-modified diacylglycerol, PEG-modified dialkylglycerol, a

mixture thereof or the like. For example, the PEGylated lipid is 1,2-dimyristoyl-rac-glycero-3-methoxy PEG (DMG-PEG2000. Other name DMG-PEG), 1,2-dipalmitoyl-rac-glycero-3-PEG (DPG-PEG), 1,2-distearoyl-rac-glycero-3-PEG (DSG-PEG), 1,2-dilauroyl-sn-glycero-3-phosphoethanolamine-PEG (DLPE-PEG), 1,2-dimyristoyl-sn-glycero-3-phosphoethanolamine-PEG (DMPE-PEG), 1,2-dipalmitoyl-sn-glycero-3-phosphocholine-PEG (DPPC-PEG) or 1, 2-distearoyl-sn-glycero-3-phosphoethanolamine-PEG (DSPE-PEG). In an aspect, the PEGylated lipid is DMG-PEG2000.

The "encapsulation efficiency" refers to the amount of the nucleic acid introduced into the lipid nanoparticles based on the total amount of the nucleic acid in the lipid nanoparticle dispersion. For example, when 98 mg of the nucleic acid of a total amount of 100 mg of the nucleic acid is introduced into the lipid nanoparticles, the encapsulation efficiency can be indicated as 98%. The "encapsulation", when used in this specification, means being completely or substantially contained in the inside or being contained. In nucleic acid lipid nanoparticles of mRNA or the like, the encapsulation efficiency of the nucleic acid is measured by the method described in detail below. The value of the encapsulation efficiency of the nucleic acid is 70% or more in an aspect, 80% or more in an aspect, 90% or more in an aspect, 93% or more in an aspect or 95% or more in an aspect.

The "N/P ratio" is a value obtained by dividing the number of moles of the amino group of the cationic lipid in the nucleic acid lipid nanoparticles (N) by the number of moles of the phosphoric acid of RNA (P). In an aspect, the N/P ratio is 1 to 12, and in an aspect, the N/P ratio is 6 to 12. In an aspect, the N/P ratio is 6 or 12, and in an aspect, the N/P ratio is 6.

"Nucleic acids" are deoxyribonucleic acid (DNA) and ribonucleic acid (RNA), and examples of the nucleic acid include messenger RNA (mRNA), microRNA (miRNA), small interfering RNA (siRNA), small hairpin RNA (shRNA), ribozymes and antisense oligonucleotides. In an aspect, the nucleic acid is mRNA. In an aspect, the nucleic acid is siRNA. In an aspect, the nucleic acid is mRNA of Ascl1 (mRNA encoding Ascl1 protein is also called Ascl mRNA, and mRNA encoding xxx protein is also called xxx mRNA below). The nucleic acid may be Dlx2 mRNA, NeuroD1 mRNA (mRNA encoding NeuroD1 protein), Ngn2 mRNA, BDNF mRNA, NGF mRNA, NT-3 mRNA or the like. In an aspect, the nucleic acid is NeuroD1 mRNA. In an aspect, the mRNA is Ascl1 mRNA. In an aspect, the nucleic acid is NeuroD1 mRNA containing a base sequence encoding a protein having the amino acid sequence of SEQ ID NO: 2. In an aspect, the nucleic acid is NeuroD1 mRNA containing the base sequence of SEQ ID NO: 1. In an aspect, the nucleic acid is NeuroD1 mRNA containing the base sequence of SEQ ID NO: 3. In an aspect, the nucleic acid is NeuroD1 mRNA containing the base sequence of SEQ ID NO: 4. In an aspect, the nucleic acid is NeuroD1 mRNA containing the base sequence of SEQ ID NO: 5. In an aspect, the nucleic acid is mRNA encoding NeuroD1 protein containing a sequence for cap addition. In an aspect, the nucleic acid is mRNA encoding NeuroD1 protein in which the 5' terminal sequence is a 5' terminal sequence suitable for the capping method using CleanCap (registered trademark) Reagent AG (TriLink BioTechnologies). In an aspect, the nucleic acid is mRNA encoding NeuroD1 protein in which the 5' terminal sequence is AGG. In an aspect, the nucleic acid is mRNA encoding NeuroD1 protein in which the 5' terminal sequence is (position 1 to position 3 of SEQ ID NO: 4). In an aspect, the nucleic acid is BDNF mRNA. In an aspect, two or more kinds of nucleic acid are contained in the lipid nanoparticles, and in an aspect, one kind of nucleic acid is contained in the lipid nanoparticles. The lipid nanoparticles encapsulating the nucleic acid contain, based on the total weight of the lipid nanoparticles, the nucleic acid in an amount of 0.001 to 60 weight% in an aspect, 0.1 to 40 weight% in an aspect, 1.0 to 25 weight% in an aspect or contains 3.0 to 10 weight% of the nucleic acid in an aspect. The mRNA may be a natural nucleic acid or an artificial nucleic acid (for example, a nucleic acid containing natural nucleic acid bases and/or artificial nucleic acid bases).

In this specification, the "mRNA encoding xxx protein" means RNA which contains a polynucleotide containing a base sequence encoding xxx protein and which can be translated to xxx protein. In this specification, mRNA encoding xxx protein is also called xxx mRNA. That is, a polynucleotide encoding xxx protein in an expressible state is included. In an aspect, the mRNA encoding xxx protein in the invention is a polynucleotide encoding xxx protein containing functional sequences for expressing xxx protein (for example, a CDS, a 5' UTR, a 3' UTR, a 5' cap structure and a poly(A) sequence are included, but the sequences are not limited thereto).

The polynucleotide in this specification is sometimes described as a base sequence containing "T" as a typical DNA sequence, but for example, when a base sequence specified by a specific SEQ ID NO: represents a DNA sequence in RNA (for example, mRNA) containing the base sequence specified by the specific SEQ ID NO:, the base sequence is understood as an RNA sequence in which the "T" in the DNA sequence are replaced with "U". The bases "adenine (A)", "thymine (T)", "guanine (G)", "cytosine (C)" and "uracil (U)" composing a base sequence (for example, the base sequences of SEQ ID NOs: 1 and 3) and nucleosides and nucleotides containing the bases may be of a natural type or a modified type for each base, each nucleoside and each nucleotide and/or have another modification (methylation or the like), unless otherwise specified in the sequence listing.

The "mRNA" is a messenger ribonucleic acid, is translated into a desired protein and contains a 5' cap, a 5' untranslated region (an untranslated region is also called UTR below), a coding region (CDS), a 3' UTR and a poly(A) chain in the structure.

The "5' cap" is a modified structure found at the 5' terminal and is involved in stability of mature mRNA, initiation of translation and the like. A structure in which 7-methylguanosine (also called $m^7G$) and mRNA are linked at 5' and 5' through triphosphoric acid (also called ppp) is called Cap-0. A structure in which position 2' of the ribose in the first nucleoside of

mRNA is methylated in addition to the Cap-0 structure is called Cap-1, and a structure in which position 2' of the riboses of the first and second nucleosides are methylated is called Cap-2. Cap-0, Cap-1 and Cap-2 are sometimes called $m^7GpppNp$, $m^7GpppN_1mp$ and $m^7GpppN_1mpN_2mp$, respectively ($N_1$ and $N_2$ each represent a nucleoside, and m represents a 2'-O methyl group) (Nature Reviews Molecular Cell Biology 2014, vol.15(5), p313-326). In an aspect, the 5' cap is Cap-0, Cap-1 or Cap-2. In an aspect, the 5' cap is Cap-0. In an aspect, the 5' cap is Cap-1. In an aspect, the 5' cap is Cap-2.

The "UTRs" are untranslated regions and include a 5' UTR and a 3' UTR. The 5' UTR and the 3' UTR may be derived from a gene to be expressed or derived from a heterologous gene. The UTRs may be of a natural type or a modified type having a sequence of a natural-type UTR which has been changed by insertion, deletion, substitution and/or addition of one or some (for example, 2, 3, 4, 5 or 6 nucleotides) nucleotides. The "UTR derived from a gene" in this specification includes not only a natural-type UTR but also such a modified-type UTR. In an UTR, more than one UTR may be linked directly or through a spacer sequence. The 5' UTR may contain a part of the Kozak sequence (for example, the sequence in the Kozak sequence at the 5' side from the start codon). In an aspect, the UTRs are α-globin gene-derived UTRs. In an aspect, the UTRs are human α-globin gene-derived UTRs. In an aspect, the UTRs are β-globin gene-derived UTRs. In an aspect, the UTRs are human β-globin gene-derived UTRs. In an aspect, the 5' UTR and the 3' UTR are human α-globin gene-derived 5' UTR and 3' UTR.

The "CDS" is a coding sequence and means a DNA sequence region translated into a protein, and the codons may be optimized. The stop codon at the 3' terminal of the CDS may be any stop codon of TAA, TGA or TAG, and more than one stop codon may be used in succession (for example, TAATGATAG).

A "modified nucleotide" is a modified nucleotide. For example, the modified nucleotide is a nucleotide modified by methylation, atom exchange, saturation of a double bond, deamination, replacement of an oxygen atom or the like with a sulfur atom or the like. In an aspect, the modified nucleotide is a nucleotide in which the nucleic acid base is modified. In an aspect, the modified nucleotide is a nucleotide in which the ribose is modified. In an aspect, the modified nucleotide is a nucleotide in which the phosphate group is modified. In an aspect, the modified nucleotide is a nucleotide containing 1-methyladenosine, pseudouridine, N1-methylpseudouridine (also called 1-methylpseudouridine), dihydrouridine, 5-methoxycytidine, 5-methylcytidine, 7-methylguanosine, N6-methyladenosine, inosine or thiouridine. In an aspect, the modified nucleotide is a nucleotide containing 1-methyladenosine. In an aspect, the modified nucleotide is a nucleotide containing modified uridine. In an aspect, the modified nucleotide is a nucleotide containing pseudouridine or N1-methylpseudouridine. In an aspect, the modified nucleotide is a nucleotide containing pseudouridine. In an aspect, the modified nucleotide is a nucleotide containing N1-methylpseudouridine. In an aspect, the modified nucleotide is a nucleotide containing dihydrouridine. In an aspect, the modified nucleotide is a nucleotide containing inosine. In an aspect, the modified nucleotide is a nucleotide containing 4-thiouridine. Here, in this specification, the modified nucleotides with "a part or all" can include those in which some of the predetermined modified nucleotides are not substituted in the entire modified nucleotide sequence. The percentage of the residues substituted to modified nucleotides from the specific nucleotides in the entire sequence may be 1 to 100%, 1 to 90%, 1 to 80%, 1 to 70%, 1 to 60%, 1 to 50%, 1 to 40%, 1 to 30%, 1 to 20%, 1 to 10%, 1 to 5%, 75 to 99%, 50 to 75%, 25 to 50% or 10 to 25%. In an aspect, "a part" as the percentage of the residues substituted to modified nucleotides of all the residues may be, for example, 60 to 99%, 70 to 99%, 80 to 99%, 90 to 99%, 95 to 99%, 60 to 99.9%, 70 to 99.9%, 80 to 99.9%, 90 to 99.9% or 95 to 99.9%.

The "poly(A) chain" is a polyadenylic acid chain having the function of stabilization of mRNA, nuclear export, translation or the like. The poly(A) chain is also called a poly(A) sequence. In an aspect, the poly(A) chain length is 20 to 1000 bases. In an aspect, the poly(A) chain length is 30 to 500 bases, 50 to 200 bases, 60 to 150 bases, 70 to 130 bases, 70 to 120 bases, 70 to 80 bases, 120 bases or 79 bases. In an aspect, the poly(A) chain length is 30 to 500 bases (in an aspect, 40 to 200, 50 to 200, 50 to 150, 50 to 100, 50 to 90, 60 to 150, 60 to 100, 60 to 90, 70 to 130, 70 to 120, 70 to 100, 70 to 90, 70 to 85, 70 to 80, 75 to 130, 75 to 120, 75 to 100 or 75 to 90, in an aspect, 74 to 84, 75 to 85, 75 to 83 or 76 to 82 bases). In an aspect, the poly(A) chain length is 120 bases. In an aspect, the poly(A) chain length is 79 bases. In an aspect, the poly(A) chain length is 74 to 84 bases. In an aspect, the poly(A) chain length is 75 to 85 bases.

The "expression" means that a polypeptide or a protein is synthesized based on the nucleotide sequence and refers to, for example, translation of mRNA into a polypeptide or a protein or synthesis of a desired protein by posttranslational modification of a polypeptide/protein.

"NeuroD1" is a neurogenic differentiation protein (neuronal differentiation 1) and is a basic helix-loop-helix (bHLH) transcription factor belonging to the NeuroD family. NeuroD1 protein activates transcription of a gene containing a specific DNA sequence known as E-box (also called transcription factor activity). NeuroD1 has a central function of inducing differentiation from neural stem cells into neurons. The glial cells are classified into four types of cells, astrocytes, oligodendrocytes, ependymal cells, and microglia.. Ectopic expression of NeuroD1 in glial cells such as NG2 cells and astrocytes is known to be able to convert glial cells into neurons (WO2014/015261A). In an aspect, NeuroD1 is a protein having the amino acid sequence of SEQ ID NO: 2. In an aspect, NeuroD1 is a protein having a sequence identity of 90% or more (for example, 95% or more, 98% or more, 99% or more, 99.4% or more or 99.5% or more) to a protein having the amino acid sequence of SEQ ID NO: 2 and which has transcription factor activity. In an aspect, NeuroD1 is a protein having

insertion, deletion, substitution and/or addition of one to 50 (for example, one or two, one to three, one to five, one to seven, one to ten or one to thirty) amino acids in the amino acid sequence of SEQ ID NO: 2 and which has transcription factor activity.

The "transcription factor activity" means the ability of a protein to activate transcription of a related gene (namely, the ability of activating transcription). Whether a protein has transcription factor activity can be determined specifically, for example, by observing whether the expression level of the related gene is increased or decreased by a recombinant vector compared to that in cells without introduction of the vector. The recombinant vector can be produced by inserting a polynucleotide encoding the protein into a vector having any promotor, and the protein can be expressed by introducing the recombinant vector into cells. For example, when the expression of the related gene is increased in cells into which a vector including a polynucleotide encoding NeuroD1 protein has been introduced, it can be determined that NeuroD1 protein has transcription factor activity.

The "sequence identity" means the percentage (%) of the matching residues between sequences when a reference biological sequence (a base sequence, an amino acid sequence or the like) and a target biological sequence are aligned (generally, the percentage % of the matching residues based on the entire length of the target sequence). The "sequence identity" can be calculated, for example, as an identity value obtained using EMBOSS Needle (Nucleic Acids Res., 2015; Vol.43: pW580-W584) using the parameters provided as default. The parameters are as follows (Gap Open Penalty = 10, Gap Extend Penalty = 0.5, Matrix = EBLOSUM62, End Gap Penalty = false).

"Penetrating branch infarction" is a disease in which infarction is caused in the thalamus, the caudate nucleus, the putamen, the globus pallidus and/or the internal capsule, which are controlled by the penetrating branch, and in which the function at the infarction site is damaged. The "penetrating branch" is a thin arteries branched from the middle cerebral artery constituting the major cerebral artery and supplies the basal ganglia/thalamus area and the internal capsule. Here, that a brain area is "territory supplied" by a vascular vessel means that blood is supplied to the region in the brain, and the region in the brain is within a vascular territory of the vascular vessel.

The "penetrating branch territory" is a region being as a territory supplied by the penetrating branch and refers to the basal ganglia and thalamic region and the internal capsule.

"Cerebral infarction having brain damage in a penetrating branch territory" means cerebral infarction in which brain damage caused by infarction is mainly caused in a penetrating branch territory. Cerebral infarction having brain damage in a penetrating branch territory includes not only cerebral infarction in which brain damage is caused in a penetrating branch territory due to infarction caused in a penetrating branch territory but also cerebral infarction in which brain damage is secondarily caused in a penetrating branch territory, for example, as in the case in which cell necrosis caused by infarction caused around a penetrating branch territory spreads to a penetrating branch territory to damage the function of the site. The brain damage in this specification means functional damage in the brain due to cell necrosis caused by infarction. The brain damage generally has cell necrosis in the area having the brain damage. "Cerebral infarction having brain damage in a penetrating branch territory" may be cerebral infarction having cell necrosis in a penetrating branch territory.

Aspects of the compound of the formula (I), which is the compound of the invention, or a salt thereof, lipid nanoparticles containing the compound or the salt and a pharmaceutical composition are shown below. In this regard, all the aspects can be freely combined when the combinations are combinations of any compatible two or more. Even when the combination is not specifically described, one aspect or two or more aspects can be combined with another aspect.

1. Cationic Lipid

(1) Aspects of the compound of the formula (I), which is the compound of the invention, or a salt thereof are shown below.

(Aspects of Cationic Lipid)

(1a) The compound of the formula (I) or a salt thereof in which $L^1$ is a $C_{6-10}$ alkylene. The compound of the formula (I) or a salt thereof in which $L^1$ is a $C_{7-9}$ alkylene. The compound of the formula (I) or a salt thereof in which $L^1$ is a $C_{6-9}$ alkylene. The compound of the formula (I) or a salt thereof in which $L^1$ is a $C_{7-8}$ alkylene. The compound of the formula (I) or a salt thereof in which $L^1$ is a $C_7$ alkylene. The compound of the formula (I) or a salt thereof in which $L^1$ is a $C_6$ alkylene. The compound of the formula (I) or a salt thereof in which $L^1$ is a $C_{9-11}$ alkylene. The compound of the formula (I) or a salt thereof in which $L^1$ is a $C_{8-12}$ alkylene. The compound of the formula (I) or a salt thereof in which $L^1$ is a $C_{7-13}$ alkylene. The compound of the formula (I) or a salt thereof in which $L^1$ is a $C_{6-14}$ alkylene. The compound of the formula

(I) or a salt thereof in which $L^1$ is a $C_{6-8}$ alkylene. The compound of the formula (I) or a salt thereof in which $L^1$ is a $C_8$ alkylene. The compound of the formula (I) or a salt thereof in which $L^1$ is a $C_9$ alkylene.

(1b) The compound of the formula (I) or a salt thereof in which $L^2$ is a $C_{6-10}$ alkyl. The compound of the formula (I) or a salt thereof in which $L^2$ is a $C_{6-8}$ alkyl. The compound of the formula (I) or a salt thereof in which $L^2$ is a $C_9$ alkyl. The compound of the formula (I) or a salt thereof in which $L^2$ is a $C_8$ alkyl. The compound of the formula (I) or a salt thereof in which $L^2$ is a $C_6$ alkyl. The compound of the formula (I) or a salt thereof in which $L^2$ is a $C_{9-11}$ alkyl. The compound of the formula (I) or a salt thereof in which $L^2$ is a $C_{8-12}$ alkyl. The compound of the formula (I) or a salt thereof in which $L^2$ is a $C_{7-13}$ alkyl. The compound of the formula (I) or a salt thereof in which $L^2$ is a $C_{6-14}$ alkyl. The compound of the formula (I) or a salt thereof in which $L^2$ is a $C_{5-15}$ alkyl. The compound of the formula (I) or a salt thereof in which $L^2$ is a $C_{10}$ alkyl. The compound of the formula (I) or a salt thereof in which $L^2$ is a $C_6$ alkyl, a $C_8$ alkyl or a $C_{10}$ alkyl.

(1c) The compound of the formula (I) or a salt thereof in which $L^3$ is a $C_{6-10}$ alkyl. The compound of the formula (I) or a salt thereof in which $L^3$ is a $C_{6-8}$ alkyl. The compound of the formula (I) or a salt thereof in which $L^3$ is a $C_8$ alkyl. The compound of the formula (I) or a salt thereof in which $L^3$ is a $C_6$ alkyl. The compound of the formula (I) or a salt thereof in which $L^3$ is a $C_6$ alkyl, a $C_8$ alkyl or a $C_{10}$ alkyl.

(1d) The compound of the formula (I) or a salt thereof in which M is -C(=O)O-. The compound of the formula (I) or a salt thereof in which M is -OC(=O)-.

(1e) The compound of the formula (I) or a salt thereof which is a combination of compatible two or more of the aspects described in (1a) to (1d) above.

Examples of the combination of (1e) are specifically the aspects below.

(2) The compound described in the formula (I) or a salt thereof in which $L^1$ is a $C_{6-9}$ alkylene, and $L^2$ and $L^3$ are each independently a $C_{6-10}$ alkyl.

(2a) The compound described in the formula (I) or a salt thereof in which $L^1$ is a $C_{6-9}$ alkylene, and $L^2$ and $L^3$ are each independently a $C_6$ alkyl, a $C_8$ alkyl or a $C_{10}$ alkyl.

(3) The compound of the formula (I) or a salt thereof in which $L^1$ is a $C_{6-9}$ alkylene, $L^2$ and $L^3$ are each independently a $C_{6-10}$ alkyl, and M is -C(=O)O-.

(3a) The compound of the formula (I) or a salt thereof in which $L^1$ is a $C_{6-8}$ alkylene, $L^2$ and $L^3$ are each independently a $C_6$ alkyl, a $C_8$ alkyl or a $C_{10}$ alkyl, and M is -C(=O)O-.

(3b) The compound of the formula (I) or a salt thereof in which $L^1$ is a $C_{6-9}$ alkylene, $L^2$ and $L^3$ are each independently a $C_{6-10}$ alkyl, and M is -OC(=O)-.

(3c) The compound of the formula (I) or a salt thereof in which $L^1$ is a $C_9$ alkylene, $L^2$ and $L^3$ are each independently a $C_6$ alkyl or a $C_8$ alkyl, and M is -OC(=O)-.

(4) Aspects of the specific compounds included in the invention are the compounds below or the salts thereof.

Di(heptadecan-9-yl) 8,8'-{[1-(2-hydroxyethyl)piperidin-4-yl]azanediyl}di(octanoate) or a salt thereof.

Di(tridecan-7-yl) 9,9'-{[1-(2-hydroxyethyl)piperidin-4-yl]azanediyl}di(nonanoate) or a salt thereof.

{[1-(2-Hydroxyethyl)piperidin-4-yl]azanediyl } di(nonane-9,1-diyl) bis(2-hexyldecanoate) or a salt thereof.

In this specification, the compound of the formula (I) or a salt thereof may be described only as one of isomers, but the invention includes isomers other than that and also includes separated isomers or mixtures thereof.

In addition, the compound of the formula (I) or a salt thereof may have an asymmetric center or an axial chirality and may have enantiomers (optical isomers) based thereon. The compound of the formula (I) or a salt thereof includes both isolated enantiomers such as (R)-form and (S)-form and mixtures thereof (including a racemic mixture or a non-racemic mixture). In an aspect, the enantiomer is "stereochemically pure". The "stereochemically pure" means the purity to a degree that one skilled in the art can recognize substantially stereochemically pure. In an aspect, the enantiomer is, for example, a compound having a stereochemical purity of 90% ee (enantiomeric excess) or more, 95% ee or more, 98% ee or more or 99% ee or more.

The salt of the compound of the formula (I) is a pharmaceutically acceptable salt and may form an acid addition salt depending on the type of the substituent. Specific examples include an acid addition salt with an inorganic acid, such as hydrochloric acid, hydrobromic acid, hydroiodic acid, sulfuric acid, nitric acid and phosphoric acid, or with an organic acid, such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid and glutamic acid, and the like.

Furthermore, the invention also includes various hydrates, solvates and crystal polymorphism substances of the compound of the formula (I) or a salt thereof.

The invention also includes the compound of the formula (I) or a salt thereof which is pharmaceutically acceptable and labeled with one or more radioactive or nonradioactive isotopes. Examples of preferable isotopes used for isotope labeling of the compound of the invention include isotopes of hydrogen ($^2H$, $^3H$ and the like), carbon ($^{11}C$, $^{13}C$, $^{14}C$ and the

like), nitrogen ($^{13}N$, $^{15}N$ and the like), oxygen ($^{15}O$, $^{17}O$, $^{18}O$ and the like), fluorine ($^{18}F$ and the like), chlorine ($^{36}Cl$ and the like), iodine ($^{123}I$, $^{125}I$ and the like), phosphorus ($^{32}P$ and the like) and sulfur ($^{35}S$ and the like). The isotopically labeled compound of the invention of the present application can be used for studies such as histological distribution study of drugs and/or substrates and the like. For example, a radioisotope such as tritium ($^{3}H$) and carbon-14 ($^{14}C$) can be used for the purpose due to easiness of labeling and convenience of detection. Replacement with a heavier isotope, for example replacement of hydrogen with deuterium ($^{2}H$), is sometimes therapeutically advantageous because metabolic stability improves (for example, increased *in vivo* half-life, decreased required dose and declined drug interaction). Replacement with a positron-emitting isotope ($^{11}C$, $^{18}F$, $^{15}O$, $^{13}N$ or the like) can be used for positron emission tomography (PET) for testing the substrate acceptor occupancy. The isotopically labeled compound of the invention can be generally produced by a conventional method known to one skilled in the art or by a production method similar to those in Examples or Production Examples or the like using an appropriate isotopically labeled reagent instead of an unlabeled reagent.

2. Lipid Nanoparticles

Aspects of lipid nanoparticles containing the compound of the formula (I), which is the compound of the invention, or a salt thereof as a cationic lipid are shown below.

(Aspects of Lipid Nanoparticles)

(5) Lipid nanoparticles containing the compound of the formula (I) or a salt thereof which contain a neutral lipid and a PEGylated lipid.
(5a) The lipid nanoparticles described in (5) in which the neutral lipid is a phospholipid and a sterol.
(5b) The lipid nanoparticles described in (5a) in which the phospholipid is DSPC.
(5c) The lipid nanoparticles described in (5a) in which the sterol is cholesterol.
(5d) The lipid nanoparticles described in (5) in which the PEGylated lipid is DMG-PEG2000.
(5e) The lipid nanoparticles described in (5) in which the composition ratio of the compound of the formula (I) or the salt thereof is 20.0 to 70.0 mol% based on the total amount of the lipid nanoparticles. Lipid nanoparticles containing the compound of the formula (I) or a salt thereof in which the composition ratio of the compound of the formula (I) or the salt thereof is 30.0 to 70.0 mol% based on the total amount of the lipid nanoparticles. The lipid nanoparticles described in (5) in which the composition ratio of the compound of the formula (I) or the salt thereof is 35.0 to 50.0 mol% based on the total amount of the lipid nanoparticles.
(5f) The lipid nanoparticles described in (5) in which the composition ratio of the neutral lipid is 27.0 to 79.5 mol% based on the total amount of the lipid nanoparticles. The lipid nanoparticles described in (5) in which the composition ratio of the neutral lipid is 28.5 to 78.5 mol% based on the total amount of the lipid nanoparticles. The lipid nanoparticles described in (5) in which the composition ratio of the neutral lipid is 27.5 to 69.0 mol% based on the total amount of the lipid nanoparticles. The lipid nanoparticles described in (5) in which the composition ratio of the neutral lipid is 47.5 to 64.0 mol% based on the total amount of the lipid nanoparticles.
(5g) The lipid nanoparticles described in (5a) in which the composition ratio of the phospholipid is 0 to 16.0 mol% based on the total amount of the lipid nanoparticles. The lipid nanoparticles described in (5a) in which the composition ratio of the phospholipid is 0 to 14.0 mol% based on the total amount of the lipid nanoparticles. The lipid nanoparticles described in (5a) in which the composition ratio of the phospholipid is 0 to 13.0 mol% based on the total amount of the lipid nanoparticles.
(5h) The lipid nanoparticles described in (5a) in which the composition ratio of the sterol is 22.5 to 62.5 mol% based on the total amount of the lipid nanoparticles. The lipid nanoparticles described in (5a) in which the composition ratio of the sterol is 22.5 to 58.5 mol% based on the total amount of the lipid nanoparticles. The lipid nanoparticles described in (5a) in which the composition ratio of the sterol is 38.5 to 58.5 mol% based on the total amount of the lipid nanoparticles.
(5i) The lipid nanoparticles described in (5) in which the composition ratio of the PEGylated lipid is 0.5 to 3.0 mol% based on the total amount of the lipid nanoparticles. The lipid nanoparticles described in (5) in which the composition ratio of the PEGylated lipid is 1.0 to 2.5 mol% based on the total amount of the lipid nanoparticles.
(6) Lipid nanoparticles containing the compound of the formula (I) or a salt thereof which encapsulate a nucleic acid. Lipid nanoparticles containing the compound of the formula (I) or a salt thereof which encapsulate a nucleic acid or particularly a nucleic acid which is useful for preventing and/or treating an astrocyte-related disease.
(7) The lipid nanoparticles described in (6) which contain a neutral lipid and a PEGylated lipid.
(7a) The lipid nanoparticles described in (7) in which the nucleic acid is mRNA. The lipid nanoparticles described in (7) in which the nucleic acid is a nucleic acid which is useful for preventing and/or treating an astrocyte-related disease.

The lipid nanoparticles described in (7) in which the nucleic acid is mRNA which is useful for preventing and/or treating an astrocyte-related disease. The lipid nanoparticles described in (7) in which the nucleic acid is Ascl1 mRNA, Dlx2 mRNA, NeuroD1 mRNA, Ngn2 mRNA, BDNF mRNA, NGF mRNA or NT-3 mRNA. The lipid nanoparticles described in (7) in which the nucleic acid is BDNF mRNA or NeuroD1 mRNA. The lipid nanoparticles described in (7) in which the nucleic acid is NeuroD1 mRNA.

(7b) The lipid nanoparticles described in (7) in which the nucleic acid is NeuroD1 mRNA containing a base sequence encoding a protein having a sequence identity of 90% or more (for example, 95% or more, 98% or more, 99% or more, 99.4% or more or 99.5% or more) to a protein having the amino acid sequence of SEQ ID NO: 2. The lipid nanoparticles described in (7) in which the nucleic acid is (i) NeuroD1 mRNA containing a base sequence encoding a protein which has the amino acid sequence of SEQ ID NO: 2 or an amino acid sequence having a sequence identity of 98% or more, preferably 99% or more, more preferably 99.4% or more or 99.5% or more, to the amino acid sequence of SEQ ID NO: 2 and which has transcription factor activity or (ii) NeuroD1 mRNA containing a base sequence encoding a protein which has an amino acid sequence having insertion, deletion, substitution and/or addition of one to 10 (for example, one or two, one to three, one to five or one to seven) amino acids in the amino acid sequence of SEQ ID NO: 2 and which has transcription factor activity. The lipid nanoparticles described in (7) in which the nucleic acid is NeuroD1 mRNA containing a base sequence having a sequence identity of 70% or more (for example, 80% or more, 90% or more or 95% or more) to the base sequence of SEQ ID NO: 1 or 3 and encodes the amino acid sequence of SEQ ID NO: 2. The lipid nanoparticles described in (7) in which the nucleic acid is NeuroD1 mRNA containing a base sequence having a sequence identity of 70% or more (for example, 80% or more, 90% or more or 95% or more) to the base sequence of SEQ ID NO: 1 and encodes the amino acid sequence of SEQ ID NO: 2. The lipid nanoparticles described in (7) in which the nucleic acid is NeuroD1 mRNA containing a base sequence having a sequence identity of 70% or more (for example, 80% or more, 90% or more or 95% or more) to the base sequence of SEQ ID NO: 3 and encodes the amino acid sequence of SEQ ID NO: 2. The lipid nanoparticles described in (7) in which the nucleic acid is NeuroD1 mRNA containing a base sequence having a sequence identity of 70% or more (for example, 80% or more, 90% or more or 95% or more) to the base sequence of positions 44 to 1114 of SEQ ID NO: 4 and encodes the amino acid sequence of SEQ ID NO: 2. The lipid nanoparticles described in (7) in which the nucleic acid is NeuroD1 mRNA containing a base sequence having a sequence identity of 70% or more (for example, 80% or more, 90% or more or 95% or more) to the base sequence of positions 44 to 1114 of SEQ ID NO: 5 and encodes the amino acid sequence of SEQ ID NO: 2.

(7c) The lipid nanoparticles described in (7) in which the nucleic acid is NeuroD1 mRNA containing a base sequence encoding a protein having the amino acid sequence of SEQ ID NO: 2. The lipid nanoparticles described in (7) in which the nucleic acid is NeuroD1 mRNA containing the base sequence of SEQ ID NO: 1. The lipid nanoparticles described in (7) in which the nucleic acid is NeuroD1 mRNA containing the base sequence of SEQ ID NO: 3. The lipid nanoparticles described in (7) in which the nucleic acid is NeuroD1 mRNA containing the base sequence of SEQ ID NO: 4. The lipid nanoparticles described in (7) in which the nucleic acid is NeuroD1 mRNA containing the base sequence of SEQ ID NO: 5.

(7d) The lipid nanoparticles described in (7) in which the nucleic acid is NeuroD1 mRNA having a 5' cap that is Cap-0, Cap-1 or Cap-2. The lipid nanoparticles described in (7) in which the nucleic acid is NeuroD1 mRNA having a 5' cap that is Cap-1.

(7e) The lipid nanoparticles described in (7) in which the nucleic acid is NeuroD1 mRNA having a 5' UTR containing the Kozak sequence. The lipid nanoparticles described in (7) in which the nucleic acid is NeuroD1 mRNA having a 5' UTR or a 3' UTR that is derived from α-globin gene. The lipid nanoparticles described in (7) in which the nucleic acid is NeuroD1 mRNA having a 5' UTR or a 3' UTR that is derived from human α-globin gene. The lipid nanoparticles described in (7) in which the nucleic acid is NeuroD 1 mRNA having a 5' UTR or a 3' UTR that is derived from β-globin gene. The lipid nanoparticles described in (7) in which the nucleic acid is NeuroD 1 mRNA having a 5' UTR or a 3' UTR that is derived from human β-globin gene. The lipid nanoparticles described in (7) in which the nucleic acid is NeuroD1 mRNA having a 5' UTR that is derived from α-globin gene. The lipid nanoparticles described in (7) in which the nucleic acid is NeuroD1 mRNA having a 5' UTR that is derived from human α-globin gene. The lipid nanoparticles described in (7) in which the nucleic acid is NeuroD1 mRNA having a 5' UTR that is derived from β-globin gene. The lipid nanoparticles described in (7) in which the nucleic acid is NeuroD1 mRNA having a 5' UTR that is derived from human β-globin gene. The lipid nanoparticles described in (7) in which the nucleic acid is NeuroD1 mRNA having a 3' UTR that is derived from human α-globin gene. The lipid nanoparticles described in (7) in which the nucleic acid is NeuroD1 mRNA having a 3' UTR that is derived from α-globin gene. The lipid nanoparticles described in (7) in which the nucleic acid is NeuroD1 mRNA having a 3' UTR that is derived from human β-globin gene.

(7f) The lipid nanoparticles described in (7) in which the nucleic acid is NeuroD1 mRNA containing a modified nucleotide. The lipid nanoparticles described in (7) in which the nucleic acid is NeuroD1 mRNA containing an N1-methylpseudouridine. The lipid nanoparticles described in (7) in which the nucleic acid is NeuroD1 mRNA in which a part or all of the uridines are N1-methylpseudouridines. The lipid nanoparticles described in (7) in which the nucleic

acid is NeuroD1 mRNA in which a part of the uridines have been substituted with N1-methylpseudouridines. The lipid nanoparticles described in (7) in which the nucleic acid is NeuroD1 mRNA in which all the uridines have been substituted with N1-methylpseudouridines.

(7g) The lipid nanoparticles described in (7) in which the nucleic acid is NeuroD1 mRNA having a poly(A) chain of 20 to 1000 bases. The lipid nanoparticles described in (7) in which the nucleic acid is NeuroD1 mRNA having a poly(A) chain of 30 to 500 bases, 50 to 200 bases, 60 to 150 bases, 70 to 130 bases, 70 to 120 bases, 70 to 80 bases, 120 bases or 79 bases. The lipid nanoparticles described in (7) in which the nucleic acid is NeuroD1 mRNA having a poly(A) chain of 120 bases. The lipid nanoparticles described in (7) in which the nucleic acid is NeuroD1 mRNA having a poly(A) chain of 79 bases.

(8) Lipid nanoparticles which are of a combination of compatible two or more of the cationic lipids (1) to (4) and the lipid nanoparticles (5) of the aspects described in (1) to (7). Lipid nanoparticles which are of a combination of compatible two or more of the cationic lipids (1) to (4) and the lipid nanoparticles (6) and (7) encapsulating a nucleic acid of the aspects described in (1) to (7).

Specific examples of the combinations of (8) above are the aspects below.

(8a) Lipid nanoparticles which encapsulate a nucleic acid and contain neutral lipids, a PEGylated lipid and the compound of the formula (I) or a salt thereof and
in which the nucleic acid is mRNA encoding NeuroD1 protein,
the compound of the formula (I) or the salt thereof is di(heptadecan-9-yl) 8,8'-{[1-(2-hydroxyethyl)piperidin-4-yl]azanediyl}di(octanoate) or a salt thereof,
the neutral lipids are DSPC and cholesterol, and
the PEGylated lipid is DMG-PEG2000.

(8b) The lipid nanoparticles described in (8a) in which the nucleic acid is mRNA encoding NeuroD1 protein containing a base sequence encoding a protein having the amino acid sequence of SEQ ID NO: 2.

(8c) The lipid nanoparticles described in (8a) in which the nucleic acid is the mRNA encoding NeuroD1 protein described in (8b) containing the base sequence of SEQ ID NO: 1.

(8d) The lipid nanoparticles described in (8a) in which the nucleic acid is the mRNA encoding NeuroD1 protein described in (8b) containing the base sequence of SEQ ID NO: 3.

(8e) The lipid nanoparticles described in (8a) in which the nucleic acid is the mRNA encoding NeuroD1 protein described in (8b) containing the base sequence of SEQ ID NO: 4.

(8f) The lipid nanoparticles described in (8a) in which the nucleic acid is the mRNA encoding NeuroD1 protein described in (8b) containing the base sequence of SEQ ID NO: 5.

(8g) Lipid nanoparticles containing 20.0 to 70.0 mol% of di(heptadecan-9-yl) 8,8'-{[1-(2-hydroxyethyl)piperidin-4-yl]azanediyl}di(octanoate) or a salt thereof, 27.0 to 79.5 mol% of DSPC and cholesterol and 0.5 to 3.0 mol% of DMG-PEG2000 based on the total amount of the lipid nanoparticles in (8a) to (8f).

(8h) Lipid nanoparticles containing 35.0 to 50.0 mol% of di(heptadecan-9-yl) 8,8'-{[1-(2-hydroxyethyl)piperidin-4-yl]azanediyl}di(octanoate) or a salt thereof, 47.5 to 64.0 mol% of DSPC and cholesterol and 1.0 to 2.5 mol% of DMG-PEG2000 based on the total amount of the lipid nanoparticles in (8a) to (8f).

(8i) Lipid nanoparticles which encapsulate a nucleic acid and contain a neutral lipid, a PEGylated lipid and the compound of the formula (I) or a salt thereof and
in which the nucleic acid is mRNA encoding NeuroD1 protein in which in the base sequence, the nucleotides at positions 1 to 3 are a 5' terminal sequence suitable for the capping method using CleanCap (registered trademark) Reagent AG (TriLink BioTechnologies), positions 4 to 43 are a 5' UTR, positions 44 to 1114 are CDS of human NeuroD1 gene (SEQ ID NO: 3), positions 1115 to 1120 are two successive stop codons, positions 1121 to 1231 are a 3' UTR, and positions 1232 to 1310 correspond to a poly(A) chain.

Aspects of the specific lipid nanoparticles included in the invention are as follows.

(9a) Lipid nanoparticles which encapsulate a nucleic acid and contain neutral lipids, a PEGylated lipid and the compound of the formula (I) or a salt thereof and
wherein the nucleic acid is mRNA encoding human ND1 having a 5' cap structure that is Cap-1, a CDS that is CDS of human NeuroD1 (here, in this specification, NeuroD1 is also called ND1) gene (SEQ ID NO: 1) and a poly(A) chain of 120 bases,
the compound of the formula (I) or the salt thereof is di(heptadecan-9-yl) 8,8'-{[1-(2-hydroxyethyl)piperidin-4-yl]azanediyl}di(octanoate) or a salt thereof,
the neutral lipids are DSPC and cholesterol, and
the PEGylated lipid is DMG-PEG2000.

(9b) Lipid nanoparticles which encapsulate a nucleic acid and contain neutral lipids, a PEGylated lipid and the compound of the formula (I) or a salt thereof and
in which the nucleic acid is mRNA encoding human ND 1 having a base sequence (SEQ ID NO: 5) in which all the uridines are N1-methylpseudouridines in a base sequence (SEQ ID NO: 4) having a 5' cap structure that is Cap-1, a 5' UTR that is derived from human α-globin gene, a CDS that is CDS of human ND1 gene (SEQ ID NO: 3), a 3' UTR that is derived from human α-globin gene and a poly(A) chain of 79 bases, where in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) sequence, and
the compound of the formula (I) or the salt thereof is di(heptadecan-9-yl) 8,8'-{[1-(2-hydroxyethyl)piperidin-4-yl]azanediyl}di(octanoate) or a salt thereof,
the neutral lipids are DSPC and cholesterol, and
the PEGylated lipid is DMG-PEG2000.
(9c) The lipid nanoparticles described in (9a) or (9b) which encapsulate a nucleic acid and contain neutral lipids, a PEGylated lipid and the compound of the formula (I) or a salt thereof and
which contain 50 mol% of di(heptadecan-9-yl) 8,8'-{[1-(2-hydroxyethyl)piperidin-4-yl]azanediyl}di(octanoate) or a salt thereof, 48.5 mol% of DSPC and cholesterol and 1.5 mol% of DMG-PEG2000 based on the total amount of the lipid nanoparticles.
(9d) The lipid nanoparticles described in (9a) or (9b) which encapsulate a nucleic acid and contain neutral lipids, a PEGylated lipid and the compound of the formula (I) or a salt thereof and
which contain 40 mol% of di(heptadecan-9-yl) 8,8'-{[1-(2-hydroxyethyl)piperidin-4-yl]azanediyl}di(octanoate) or a salt thereof, 58.5 mol% of DSPC and cholesterol and 1.5 mol% of DMG-PEG2000 based on the total amount of the lipid nanoparticles in (9a) to (9b).

3. Pharmaceutical Composition

Aspects of the pharmaceutical composition containing lipid nanoparticles containing the compound of the formula (I), which is the compound of the invention, or a salt thereof and one or more pharmaceutically acceptable pharmaceutical additives are shown below.

(Aspects of Pharmaceutical Composition)

(10a) A pharmaceutical composition containing the lipid nanoparticles described in any of (5) to (5i), (6), (7) to (7g), (8a) to (8i) and (9a) to (9d) and one or more pharmaceutically acceptable pharmaceutical additives.
(10b) The pharmaceutical composition described in (10a) for the prevention or the treatment of an astrocyte-related disease.
(10c) A pharmaceutical composition containing the lipid nanoparticles described in (8a) and one or more pharmaceutically acceptable pharmaceutical additives.
(10d) A pharmaceutical composition containing the lipid nanoparticles described in (8b) and one or more pharmaceutically acceptable pharmaceutical additives.

Aspects of the specific pharmaceutical compositions included in the invention are as follows.

(10e) A pharmaceutical composition containing lipid nanoparticles which encapsulate a nucleic acid and contain neutral lipids, a PEGylated lipid and the compound of the formula (I) or a salt thereof and
wherein the nucleic acid is mRNA encoding NeuroD1 protein,
the compound of the formula (I) or the salt thereof is di(heptadecan-9-yl) 8,8'-{[1-(2-hydroxyethyl)piperidin-4-yl]azanediyl}di(octanoate) or a salt thereof, the neutral lipids are DSPC and cholesterol and the PEGylated lipid is DMG-PEG2000.
(10f) A pharmaceutical composition containing lipid nanoparticles which encapsulate a nucleic acid and contain neutral lipids, a PEGylated lipid and the compound of the formula (I) or a salt thereof and
wherein the nucleic acid is mRNA encoding NeuroD1 protein containing a base sequence encoding a protein having the amino acid sequence of SEQ ID NO: 2, and
the compound of the formula (I) or the salt thereof is di(heptadecan-9-yl) 8,8'-{[1-(2-hydroxyethyl)piperidin-4-yl]azanediyl}di(octanoate) or a salt thereof,

the neutral lipids are DSPC and cholesterol and
the PEGylated lipid is DMG-PEG2000.

(10g) A pharmaceutical composition containing lipid nanoparticles which encapsulate a nucleic acid and contain neutral lipids, a PEGylated lipid and the compound of the formula (I) or a salt thereof and
in which the nucleic acid is mRNA encoding human ND 1 having a 5' cap structure that is Cap-1, a CDS that is CDS of human ND1 gene (SEQ ID NO: 1) and a poly(A) chain of 120 bases, and
where lipid nanoparticles in which the compound of the formula (I) or the salt thereof is di(heptadecan-9-yl) 8,8'-{[1-(2-hydroxyethyl)piperidin-4-yl]azanediyl}di(octanoate) or a salt thereof,
the neutral lipids are DSPC and cholesterol and
the PEGylated lipid is DMG-PEG2000.

(10h) A pharmaceutical composition containing lipid nanoparticles which encapsulate a nucleic acid and contain neutral lipids, a PEGylated lipid and the compound of the formula (I) or a salt thereof and
in which the nucleic acid is mRNA encoding human ND1 having a base sequence (SEQ ID NO: 5) in which all the uridines are N1-methylpseudouridines in a base sequence (SEQ ID NO: 4) having a 5' cap structure that is Cap-1, a 5' UTR that is derived from human α-globin gene, a CDS that is CDS of human ND1 gene (SEQ ID NO: 3), a 3' UTR that is derived from human α-globin gene and a poly(A) chain containing 79 bases, where in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) sequence,
where lipid nanoparticles in which the compound of the formula (I) or the salt thereof is di(heptadecan-9-yl) 8,8'-{[1-(2-hydroxyethyl)piperidin-4-yl]azanediyl}di(octanoate) or a salt thereof,
the neutral lipids are DSPC and cholesterol and
the PEGylated lipid is DMG-PEG2000.

Although the administration form of the pharmaceutical composition is not limited, examples thereof include parenteral administration by intracerebral, intraarticular, intravenous, intramuscular, subcutaneous or other injection, a transmucosal liquid agent, an inhalant or the like. In general, through intracerebral administration, the pharmaceutical composition is delivered to the central nervous system. In an aspect, the administration is administration into the brain parenchyma. In an aspect, the administration is intraventricular administration. In an aspect, the administration is intraspinal administration.. In an aspect, the administration is intrathecal administration. In an aspect, the administration is administration into the spinal cord parenchyma.

In general, in the case of intracerebral administration, the pharmaceutical composition containing the lipid nanoparticles of the invention is administered at about 0.001 to 10 mg per 1 kg of the brain in an aspect, at about 0.001 to 50 mg per 1 kg of the brain in an aspect or about 0.001 to 100 mg per 1 kg of the brain in an aspect, once a day or in several divided portions. The dose is appropriately decided depending on the individual case considering the disease, the symptom, the age, the gender and the like.

In general, in the case of intramuscular administration, the daily dose of the pharmaceutical composition of the invention is appropriately about 0.00001 to 10 mg/kg per body weight, and the administration is once a day or in several divided portions. The dose is appropriately decided depending on the individual case considering the disease, the symptom, the age, the gender and the like.

Although it depends on the route of administration, the dosage form, the site of administration and the types of the excipient and the additive, the pharmaceutical composition of the invention contains, based on the total weight of the pharmaceutical composition, 5 to 50 weight% of the lipid nanoparticles in an aspect, and the amount of the lipid nanoparticles or the like is 3 to 70 weight% in an aspect or 10 to 50 weight% in an aspect.

The pharmaceutical composition of the invention can be used in combination with various therapeutic agents or preventive agents for a disease to which the pharmaceutical composition is considered to have an effectiveness. The combination use may be simultaneous administration or separate administration which is sequential or at a desired interval. A simultaneous administration preparation may be a formulated agent or may be separately formulated.

4. Production Method of Cationic Lipid

The compound of the formula (I), which is the compound of the invention, or a salt thereof can be produced by applying various known synthetic methods using characteristics based on the basic structure or the type of substituent thereof. Here, depending on the type of the functional group, it is sometimes effective as a production technique to substitute the functional group with an appropriate protective group (a group that can be easily converted to the functional group) in the process from a raw material to an intermediate. Examples of the protective group include protective groups described in Wuts (P. G. M. Wuts) and Greene (T. W. Greene), "Greene's Protective Groups in Organic Synthesis (5th

edition, 2014)" and the like, and a group may be appropriately selected and used depending on the reaction conditions. In such a method, a desired compound can be obtained by conducting a reaction with the protective group introduced and then removing the protective group, when required.

Typical methods for producing the compound of the formula (I) or a salt thereof are explained below. The production methods can also be carried out referring to the references attached to the descriptions. In this regard, the production method of the invention is not limited to the examples shown below.

(First Production Method)

The compound of the invention or a salt thereof can be produced by the production methods below.

[Chem. 4]

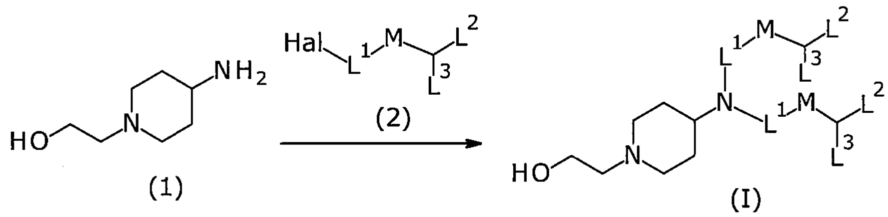

(In the formula, Hal represents a halogen.)

The compound (I) of the invention can be produced by a reaction of a raw material compound (1) and a raw material compound (2). In this reaction, the compound (1) and the compound (2) are used in an equal amount or with the compound (2) in an excess amount, and the mixture thereof is reacted in a solvent inert to the reaction or without solvent, from under cooling to under reflux with heat, at 0°C to 80°C in an aspect, at 60°C to 100°C in an aspect or at 100 to 160°C using microwaves in an aspect and stirred generally for 0.1 hours to five days. Examples of the solvent include aromatic hydrocarbons such as benzene, toluene and xylene, ethers such as diethyl ether, THF, dioxane, dimethoxyethane and cyclopentyl methyl ether, halogenated hydrocarbons such as dichloromethane, 1,2-dichloroethane and chloroform, DMF, DMSO, ethyl acetate, acetonitrile, N,N-dimethylacetamide and a mixture thereof. The reaction sometimes progresses in the presence of an organic base such as TEA, DIPEA and N-methylmorpholine or an inorganic base such as potassium carbonate, sodium carbonate and potassium hydroxide. The reaction sometimes progresses in the presence of an inorganic salt such as potassium iodide.

[Reference] S. R. Sandler and W. Karo, "Organic Functional Group Preparations", 2nd edition, Vol. 1, Academic Press Inc., 1991 and "Jikken Kagaku Koza (Courses in Experimental Chemistry) (5th edition)" edited by The Chemical Society of Japan, Vol. 14 (2005) (Maruzen)

(Raw Material First Production Method)

[Chem. 5]

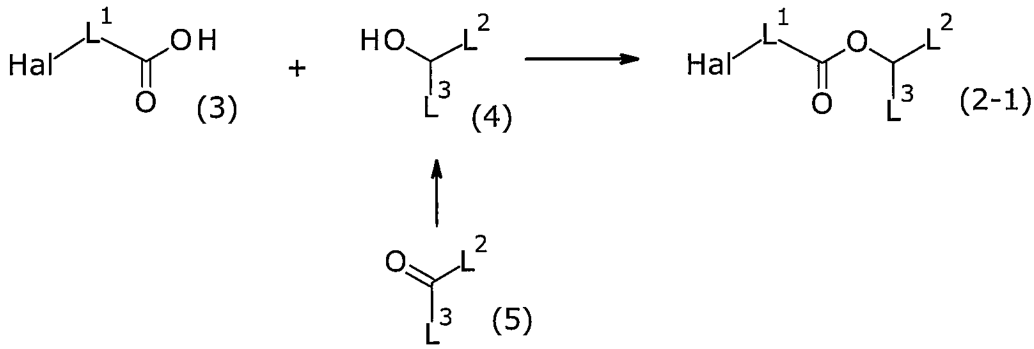

(In the formulae, Hal represents a halogen.)

A compound (2-1) is the compound (2) in which M is -C(=O)O- and can be produced by a reaction of a compound

(3) and a compound (4).

In this reaction, the compound (3) and the compound (4) are used in an equal amount or with either thereof in an excess amount, and the mixture thereof is stirred in the presence of a condensing agent, in a solvent inert to the reaction, under cooling to under heating, or at -20°C to 60°C in an aspect, generally for 0.1 hours to five days. Examples of the solvent include aromatic hydrocarbons such as benzene, toluene and xylene, halogenated hydrocarbons such as dichloromethane, 1,2-dichloroethane and chloroform, ethers such as THF, diethyl ether, dioxane and dimethoxyethane, DMF, DMSO, acetonitrile and a mixture thereof. Examples of the condensing agent include 1-(3-dimethylaminopropyl)-3-ethylcarbodiimide, 1-(3-dimethylaminopropyl)-3-ethylcarbodiimide hydrochloride, dicyclohexylcarbodiimide, 1,1'-carbonyldiimidazole, diphenylphosphoryl azide, phosphorus oxychloride and the like. An additive (for example, DMAP or 1-hydroxybenzotriazole) sometimes progresses the reaction. The reaction sometimes progresses in the presence of an organic base such as TEA, DIPEA and N-methylmorpholine.

A method in which the carboxylic acid (3) is converted to a reactive derivative and then reacted with the compound (4) can also be used. Examples of the reactive derivative of the carboxylic acid (3) include an acid halogenation product obtained by a reaction with a halogenating agent such as phosphorus oxychloride and thionyl chloride, a mixed acid anhydride obtained by a reaction with isobutyl chloroformate or the like, an active ester obtained by condensation with 1-hydroxybenzotriazole or the like and the like. The reaction of such a reactive derivative and the compound (4) can be performed in a solvent inert to the reaction such as a halogenated hydrocarbon, an aromatic hydrocarbon and an ether, from under cooling to under heating, or at -20°C to 60°C in an aspect.

[Reference] S. R. Sandler and W. Karo, "Organic Functional Group Preparations", 2nd edition, Vol. 1, Academic Press Inc., 1991 and "Jikken Kagaku Koza (Courses in Experimental Chemistry) (5th edition)" edited by The Chemical Society of Japan, Vol. 16 (2005) (Maruzen)

The compound (4) can be produced by a reduction reaction of a compound (5). In this reaction, the compound (5) is treated with an equal amount or an excess amount of a reducing agent in a solvent inert to the reaction, under cooling to under heating, or at -20°C to 80°C in an aspect, generally for 0.1 hours to three days. Examples of the solvent include ethers such as diethyl ether, THF, dioxane and dimethoxyethane, alcohols such as methanol, ethanol and 2-propanol, aromatic hydrocarbons such as toluene and xylene, DMF, DMSO, ethyl acetate and a mixture thereof. As the reducing agent, a hydride reducing agent such as sodium borohydride or diisobutylaluminum hydride, a metal reducing agent such as sodium, zinc and iron or another reducing agent in the references below is preferably used.

[Reference] M. Hudlicky, "Reductions in Organic Chemistry, 2nd ed (ACS Monograph: 188)", ACS, 1996, R. C. Larock, "Comprehensive Organic Transformations", 2nd edition, VCH Publishers, Inc., 1999, T. J. Donohoe, "Oxidation and Reduction in Organic Synthesis (Oxford Chemistry Primers 6)", Oxford Science Publications, 2000 and "Jikken Kagaku Koza (Courses in Experimental Chemistry) (5th edition)" edited by The Chemical Society of Japan, Vol. 14 (2005) (Maruzen)

(Raw Material Second Production Method)

[Chem. 6]

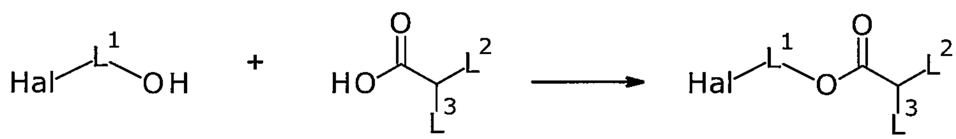

(6) (7) (2-2)

(In the formulae, Hal represents a halogen.)

A compound (2-2) is the compound (2) in which M is -O-C(=O)- and can be produced from a compound (6) and a compound (7). For this production method, a similar method to the production method for producing the compound (2-1) from the compound (3) and the compound (4) can be used.

The compound of the formula (I) or a salt thereof is isolated and purified as a free compound or a salt, a hydrate, a solvate or a crystal polymorphous substance thereof. The compound of the formula (I) or a salt thereof can also be produced by subjecting to a salt formation reaction which is an ordinary method. The isolation and purification are performed by applying a general operation, such as extraction, fractional crystallization and various types of fraction chromatography (silica gel chromatography and the like).

Various isomers can be produced by selecting an appropriate raw material compound or can be separated using a difference in physicochemical properties between the isomers. For example, an optical isomer can be obtained by a general optical resolution method of a racemate (for example, fractional crystallization for inducing to a diastereomer salt with an optically active base or acid, chromatography using a chiral column or the like and the like) and can also be produced from an appropriate optically active raw material compound.

5. Production Method of Lipid Nanoparticles

The lipid nanoparticles can be produced by adding a component such as a nucleic acid to components of the compound of the formula (I) or a salt thereof, a neutral lipid and a PEGylated lipid and dispersing in a medium.
For example, the compound of the formula (I) or a salt thereof, DSPC, a sterol, a PEGylated lipid and the like are dissolved in a solvent to obtain an oil phase, and the oil phase is mixed with or suspended in an aqueous phase such as a buffer solution containing a nucleic acid. Then, the solvent in the mixture solution is removed by a method such as dialysis and ultrafiltration, and lipid nanoparticles can be thus obtained.
Moreover, a pharmaceutical composition can be produced by a known method using an additive such as the excipient described above and the nucleic acid lipid nanoparticles.

6. Production Method of Pharmaceutical Composition

The pharmaceutical composition can be prepared by a generally used method using a pharmaceutical additive (an excipient or the like) generally used in the field, namely, a pharmaceutical excipient or the like. Regarding the pharmaceutical additive, although the components contained in the pharmaceutical composition are not limited, an additive, such as a preservative, a stabilizer, an antioxidant and an antiseptic, may be contained in addition to the excipient. Another additive may be added to the pharmaceutical composition.
Moreover, a pharmaceutically acceptable medium may be added to the pharmaceutical composition.
The pharmaceutical composition is refrigerated or frozen for storage and/or transport. The temperature is, in an aspect, about -150°C to about 0°C, in an aspect, about -80°C to about -20°C, about -40°C to about -20°C, or in an aspect, 4°C or lower. In an aspect, the solution is PBS.
The injection preferably contains a sterile aqueous or nonaqueous solution, suspension or emulsion. Examples of the aqueous solvent include distilled water for injection or physiological saline. An example of the nonaqueous solvent is an alcohol such as ethanol. Such a composition may further contain an isotonizing agent, an antiseptic, a wetting agent, an emulsifier, a dispersant, a stabilizer or a dissolution aid. These are sterilized, for example, by filtration through a bacteria keeping filter, incorporation of a microbicide or irradiation. In addition, such a composition can be produced as a sterile solid composition which is dissolved or suspended in sterile water or a sterile solvent for injection before use.
The transmucosal agent such as an inhalant or a transnasal agent is used in a liquid form and can be produced according to a conventionally known method. For example, a known excipient and in addition, a pH modifier, an antiseptic, a surfactant, a lubricant, a stabilizer, a thickener or the like may be appropriately added. The administration can be performed using an appropriate device for inhalation or insufflation. For example, the agent can be administered using a known device such as a metering and administering inhalation device or an atomizer, as a compound alone or a powder of a formulated mixture, or as a solution or a suspension in combination with a pharmaceutically acceptable pharmaceutical additive. A dry powder inhaler or the like may be for a single administration or multiple administrations. The agent may be used in a form of a pressurized aerosol spray or the like using an appropriate ejection agent, for example, a suitable gas, such as a chlorofluoroalkane or carbon dioxide.

7. Production Method of Nucleic Acid

The nucleic acid, such as mRNA for example, can be produced using a known technique in the field. Examples are shown below, but the production is not limited to the examples. mRNA can be produced using a linear plasmid as a DNA template by *in vitro* transcription (IVT). As the production methods, (i) the post-transcriptional capping method (in the process, plasmid production, *in vitro* transcription and 5' capping are conducted in this order), (ii) the co-transcriptional capping method (in the process, plasmid production and then *in vitro* transcription together with 5' capping are conducted) and the like are known. In the co-transcriptional capping method, Cap-0 is added to the 5' terminal of the RNA when the anti-reverse cap analog (ARCA) technique is used, and Cap-1 can be added to the 5' terminal of the RNA when the CleanCap (registered trademark, TriLink) capping technique is used.

(*in vitro* Transcription)

In IVT, a transcription buffer solution, nucleoside triphosphates (NTPs), an RNase inhibitor and a polymerase

(example: T7 RNA polymerase) can be generally used for the reaction. The NTPs may be of natural type or unnatural type (modified type).

(Capping)

The capping can be conducted according to a method using vaccinia capping enzyme, 2'O-methyltransferase, CleanCap or the like or another method. When the sequence transcribed by IVT does not contain any poly(A) sequence, poly(A) addition reaction can also be conducted. Here, regarding the sequence, when the capping method using CleanCap (registered trademark) Reagent AG (TriLink BioTechnologies) is used, the 5' terminal sequence suitable for the capping method using CleanCap (registered trademark) Reagent AG (TriLink BioTechnologies) is, for example, AGG (position 1 to position 3 of SEQ ID NO: 4).

(Plasmid Production)

The linear plasmid can be produced by the following processes. A plasmid obtained by inserting an UTR and a base sequence (example: a base sequence encoding NeuroD1 protein) into a plasmid (example: a high-copy plasmid for *Escherichia coli* (pUC18, pCU18 or the like)) can be produced and can be amplified using *Escherichia coli* or the like. After purification, the linear plasmid can be produced from the plasmid using a restriction enzyme and a buffer solution. After purification, the linear plasmid can be used directly as a DNA template. Alternatively, the linear plasmid can be used as a DNA template after subjecting to polymerase chain reaction (PCR).

(Purification)

The mRNA and the intermediates in the production processes can be purified by a known method in the field. Examples are shown below, but the purification is not limited to the examples. The DNA template can be removed with deoxyribonuclease I (DNase I). The transcribed RNA can be purified using a silica gel column or the like. The RNA containing a poly(A) sequence can be purified with an Oligo dT column. The linear plasmid can be purified using PureLink (registered trademark) (Thermo Fisher Scientific) or the like.

Test Examples

The pharmacological activities of nucleic acid lipid nanoparticles composed of the compounds of the formula (I) or salts thereof were examined by the following tests. In the Test Examples and the like below in this specification, the abbreviations below are sometimes used.

(Abbreviations)

B-27: B-27 serum-free supplement, BDNF: brain-derived neurotrophic factor, DMEM: Dulbecco's modified Eagle medium, eGFP: enhanced green fluorescent protein, eGFP nucleic acid lipid nanoparticles: lipid nanoparticles encapsulating eGFP mRNA, FLuc nucleic acid lipid nanoparticles: lipid nanoparticles encapsulating FLuc mRNA, ND 1 nucleic acid lipid nanoparticles: lipid nanoparticles encapsulating NeuroD1 mRNA, FBS: fetal bovine serum, FLuc: luciferase (firefly), LNP: lipid nanoparticles, mRNA: messenger ribonucleic acid, ND1: NeuroD1, PBS: phosphate-buffered saline, PFA: paraformaldehyde, RLU: relative light unit, and RGFP: relative GFP expression level.

(Evaluation of Introduction of Lipid Nanoparticles Using Mouse Primary Astrocytes)

By evaluating the efficiencies of introduction of nucleic acid (mRNA) lipid nanoparticles to mouse primary astrocytes, the effects of the compounds of the formula (I) or salts thereof composing lipid nanoparticles were evaluated. As the mRNA, FLuc mRNA and eGFP mRNA (TriLink BioTechnologies) were used, and nucleic acid lipid nanoparticles were produced using NanoAssemblr (Precision NanoSystems). The efficiencies of introduction of the test drugs (nucleic acid lipid nanoparticles) were calculated by evaluating the expression levels of luciferase and eGFP encoded by the mRNA. Here, three wells were used for each group. The methods and the results are shown in Test Examples 1 and 2.

Test Example 1: *in vitro* Luciferase Assay

(Acquisition of Mouse Primary Astrocytes)

Mouse primary astrocytes were acquired in accordance with Lynette C. Foo., Purification of Rat and Mouse

Astrocytes by Immunopanning, Cold Spring Harbor Protocols, 2013, vol.5, p.421-432. As the mouse, newborn P1-P10 of C57BL/6J (Charles River Laboratories Japan, Inc.) was used. In this regard, however, the cells were recovered in 5% $CO_2$.

(Test Method)

The mouse primary astrocytes acquired by the above method were seeded at 2000 cells/well in a poly-D-lysine coated 96-well plate (IWAKI) and cultured in 5% $CO_2$ at 37°C overnight. As the medium, 100 μL/well of DMEM/F-12 medium (Thermo Fisher Scientific) containing 2% B-27 (registered trademark) supplement (Thermo Fisher Scientific), 10% FBS (Hyclone) and 1% penicillin-streptomycin (PS) (Thermo Fisher Scientific) was used. On the next day, dispersions of the test drugs (FLuc nucleic acid lipid nanoparticles) diluted with PBS were diluted with the medium, and 100 μL thereof were added to a final concentration of 0.8 μg/mL. The cells were cultured at 200 μL/well overnight. After removing all the medium on the next day, ONE-Glo Luciferase assay system (Promega) was diluted two-fold with PBS, and 100 μL thereof was added to each well. After mixing for a minute, 80 μL thereof were transferred to a 96-well white plate (Nunc), and the luminescence thereof was detected with Envision (Perkin Elmer). RLU was used as the indicator of activity.

(Evaluation of Expression)

As a result of the above test, luciferase activity was observed in the nucleic acid lipid nanoparticles containing typical Example compounds of the invention as a component. This suggests that the lipid nanoparticles encapsulating FLuc mRNA were introduced into the astrocytes, namely the cells, and translated, and thus luciferase protein was expressed. The results of the nucleic acid lipid nanoparticles containing the Example compounds are shown in the table below. In the table, the average RLU values of the groups are shown. Ex1-L0 to Ex5-L0 shown in NUM in the table below indicate Ex1-L0-FLuc mRNA to Ex5-L0-FLuc mRNA, respectively. This means that Ex1-L0 to Ex5-L0 are lipid nanoparticles each encapsulating FLuc mRNA, having the same lipid composition as that of Ex1-L0 (cationic lipid | DSPC | cholesterol | DMG-PEG2000 = 50 | 10 | 38.5 | 1.5 (mol%)) and containing the Example compounds Ex1, Ex2, Ex3, Ex4 and Ex5, respectively.

[Table 1]

| NUM | RLU |
|---|---|
| Ex1- L0 | 10645733 |
| Ex2-L0 | 4290933 |
| Ex3-L0 | 3928667 |
| Ex4-L0 | 10920800 |
| Ex5-L0 | 10392533 |

Test Example 2: LNP Assay Using *in vitro* eGFP

(Test Method)

The mouse primary astrocytes acquired by the above method were seeded at 10,000 cells/well in a poly-D-lysine coated 96-well plate (Corning) and cultured in 5% $CO_2$ at 37°C overnight. As the medium, 200 μL/well of DMEM/F-12 medium (Thermo Fisher Scientific) containing 2% B-27 (registered trademark) supplement (Thermo Fisher Scientific), 10% FBS (Cytiva) and 1% penicillin-streptomycin (PS) (Thermo Fisher Scientific) was used. On the next day, a half the amount of the medium (100 μL) was removed, and 100 μL of the test drugs (eGFP nucleic acid lipid nanoparticles) which were diluted with the medium to a concentration of 1.6 μg/mL were added to a final concentration of 0.8 μg/mL. The cells were cultured overnight. All the medium was removed on the next day, and the plate was left still for 10 minutes in 50 μL of 4% PFA (WAKO) to fix the cells. Then, the cells were washed with PBS (WAKO), and 100 μL of Hoechst (Thermo Fisher Scientific) diluted 1000-fold with PBS was added. The plate was left still for five minutes. Then, the cells were washed twice with PBS, and after adding 150 μL of PBS, the expression intensities of eGFP were detected in this state using IN Cell Analyzer 6000 (Cytiva) as fluorescent signals. Moreover, the cell nuclei were detected as the fluorescent signals of Hoechst. The value obtained by dividing the fluorescent intensity of a well by the area of the cell nucleus was used as the indicator of activity.

(Evaluation of Expression)

As a result of the above test, fluorescence of eGFP was observed in the nucleic acid lipid nanoparticles containing typical Example compound Ex1 of the invention (Ex1-L1 to Ex1-L17). This suggests that eGFP mRNA was introduced into the cells with the lipid nanoparticles containing the Example lipid as a component and translated, and thus eGFP protein was expressed. The average indicators of activity of the groups of the expression of the nucleic acid lipid nanoparticles were calculated, and the relative values based on that of Ex1-L1 regarded as 1 are shown as RGFP in the table below. Ex1-L1 to Ex1-L17 shown in NUM in the table below indicate Ex1-L1-eGFP mRNA to Ex1-L17-eGFP mRNA, respectively. This means that Ex1-L1 to Ex1-L17 are lipid nanoparticles each encapsulating eGFP mRNA, having the lipid composition described in Example 10 below and containing the Example compound Ex1.

[Table 2]

| NUM | RGFP |
|---|---|
| Ex1-L1 | 1.00 |
| Ex1-L2 | 0.13 |
| Ex1-L3 | 0.66 |
| Ex1-L4 | 0.81 |
| Ex1-L5 | 4.63 |
| Ex1-L6 | 2.56 |
| Ex1-L7 | 0.96 |
| Ex1-L8 | 1.15 |
| Ex1-L9 | 2.13 |
| | |
| NUM | RGFP |
| Ex1-L10 | 5.82 |
| Ex1-L11 | 0.21 |
| Ex1-L12 | 2.90 |
| Ex1-L13 | 3.73 |
| Ex1-L14 | 2.12 |
| Ex1-L15 | 0.59 |
| Ex1-L16 | 2.24 |
| Ex1-L17 | 2.08 |

Test Example 3: *in vivo* Luciferase Assay

(Test Method)

The efficiencies of introduction of nucleic acid lipid nanoparticles in the brain were evaluated using IVIS spectrum (PerkinElmer). Nucleic acid lipid nanoparticles were administered into the brain parenchyma of 6-12-week-old BALB/c mice (Charles River Laboratories Japan, Inc., n=3) under isoflurane (isoflurane inhalation anesthetic solution "VTRS"; Mylan N.V., the same applies below) anesthesia. For the administration into the brain parenchyma, the coordinates for administration were determined at 0.0 mm anterior-posterior from the bregma, 2.0 mm to the left and 2.5 mm deep based on the mouse brain atlas (Academic press). Under isoflurane anesthesia, after shaving with clippers, the skull was fixed with a brain stereotaxic apparatus (KOPF), and a hole with a diameter of around 1 mm was made with a drill (Foredom). An Ito syringe (Ito Corporation) was filled with a test drug (FLuc nucleic acid lipid nanoparticles) of 0.3 mg/mL, and 1 μL thereof was administered to the set coordinates using an injection pump (Narishige Group) at a flow rate of 0.2 μL/min. After leaving still for a minute after the administration, the needle was pulled out slowly, and the scalp was stitched together. After a day, 250 μL/animal of 15 mg/mL luciferin (Promega) was intraperitoneally administered under isoflurane anesthesia, and the luminescence was observed after 20 minutes using IVIS spectrum. The obtained luminescence values were

converted to digits with photons/sec unit with Living image software and used for the analysis. (In addition, for example, the nucleic acid lipid nanoparticles of a representative example of the present invention were also compared with nucleic acid lipid nanoparticles in which the example compound was replaced with the reference compound described in [Chem. 1]. The reference compound was produced according to the method disclosed therein).

(Evaluation of Expression)

As a result of the above test, luciferase activity was observed with the nucleic acid lipid nanoparticles of a typical Example of the invention (Ex1-L01-Fluc mRNA). This suggests that the lipid nanoparticles encapsulating FLuc mRNA were introduced into the cells in the brain and translated, and thus luciferase protein was expressed. The data of expression of the nucleic acid lipid nanoparticles are shown in the table below. In the table, the average of the three individuals of Ex1-L01-Fluc mRNA evaluated is shown. Ex1-L01 shown in NUM in the table below indicates Ex1-L01-FLuc mRNA. This means that Ex1-L01 is lipid nanoparticles encapsulating FLuc mRNA, having the same lipid composition as that of Ex1-L0 and containing the Example compound Ex1.

[Table 3]

| NUM | Bioluminescence imaging (photons/sec) |
|---|---|
| Ex1-L01 | 129900000.00 |

Test Example 4: *in vitro* Conversion of Rat Primary Astrocytes into Neurons

(Addition of Nucleic Acid Lipid Nanoparticles to Rat Primary Astrocytes)

Rat primary astrocytes were acquired in accordance with Lynette C. Foo., Purification of Rat and Mouse Astrocytes by Immunopanning, Cold Spring Harbor Protocols, 2013, vol.5, p.421-432 in the same manner as that described in Test Example 1 and used. As the rats, newborn Wistar rats (CLEA Japan, Inc., P1-P10) were used. In this regard, however, the cells were recovered in 5% $CO_2$. The acquired rat primary astrocytes were seeded at 10,000 to 40,000 cells/well in a poly-D-lysine coated 96-well plate (Corning, 356640) and cultured in 5% $CO_2$ at 37°C overnight. For the culture, 200 μL/well of DMEM/F-12 medium (Thermo Fisher Scientific, 11320-033) containing 2% B-27 (registered trademark) supplement (Thermo Fisher Scientific, A1895601), 10% fetal bovine serum (FBS, Cytiva, SH30084.03) and 1% penicillin-streptomycin (PS) (Thermo Fisher Scientific, 15070063) was used. The DMEM/F-12 medium containing B-27 (registered trademark) supplement, FBS and PS was used as the culture medium also below. On the next day, the dispersions of ND1 nucleic acid lipid nanoparticles (EX1-L1-ND1-1 or EX1-L6-ND1-2) diluted with PBS, which were prepared in Example 10 below, were diluted with the culture medium and added to a final concentration of 0.5 μg/mL, and the cells were cultured for 24 hours. PBS and the culture medium were added to the control. Three wells were used for each group.

(Examination of Expression of ND1 Protein)

The culture medium was removed 24 hours after the addition of the ND1 nucleic acid lipid nanoparticles, and the cells were fixed with 4% paraformaldehyde. Then, the cells were washed with PBS and immunostained. The expression of ND1 protein was examined with an anti-ND1 antibody (Santa Cruz Biotechnology, sc46684), and it was shown that ND1 protein-positive cells significantly increased in the astrocytes to which Ex1-L1-ND1-1 or Ex1-L6-ND1-2 was added, compared to those in the astrocytes to which PBS was added (control).

(Examination of Expression of Neuron Marker)

The culture medium was removed 24 hours after the addition of the ND1 nucleic acid lipid nanoparticles and changed to a transdifferentiation medium, and the medium was changed every two to three days. The transdifferentiation medium was DMEM/F-12 medium containing 2% B-27 (registered trademark) supplement, 1% GlutaMAX (trademark) supplement (Thermo Fisher Scientific, 35050-061), 1% PS and 0.02% BDNF solution, and 200 μL/well of the medium was added. The BDNF solution was prepared by dissolving BDNF powder (PeproTech, 450-02) in pure water to be 100 μg/mL. All the transdifferentiation medium was removed on the sixth to seventh day after the addition of the ND1 nucleic acid lipid nanoparticles, and the cells were fixed with 4% paraformaldehyde. Then, the cells were washed with PBS, and the cells were immunostained using an anti-DCX antibody (Cell Signaling Technology, 4604S) to examine the expression of Doublecortin (DCX), which is a marker of immature neurons.

It was found that DCX was expressed significantly in the astrocytes to which Ex1-L1-ND1-1 or Ex1-L6-ND1-2 was added, compared to that in the astrocytes to which PBS was added (control). The results suggest that the rat primary astrocytes were converted to neurons by ND1 mRNA.

Test Example 5: Action of ND 1 Nucleic Acid Lipid Nanoparticles on Cynomolgus Monkey Cerebral Infarction Model

(Production of Cynomolgus Monkey Cerebral Infarction Model)

Four individuals of male cynomolgus monkey (three years old or older, Shin Nippon Biomedical Laboratories, Ltd.) were subjected to induction anesthesia by intramuscular injection (i.m.) using ketamine hydrochloride (Ketalar for muscular injection 500 mg; Daiichi Sankyo Propharma Co., Ltd.) in an amount of about 10 mg/kg. The cynomolgus monkeys under anesthesia were intratracheally intubated and subjected to maintenance anesthesia by inhalation of isoflurane (isoflurane inhalation anesthetic solution "VTRS"; Mylan N.V.) through the tracheal tube under mechanical ventilation or with spontaneous breathing. After the hair on the head of each cynomolgus monkey under anesthesia was shaved with clippers, the cynomolgus monkey was fixed with a brain stereotaxic apparatus (Narishige Group). Then, with consideration for bleeding, the skin in the head of the cynomolgus monkey was cut open, and the bregma, which is the intersection of the sagittal suture and the coronal suture in the front of the skull surface, was observed. The infusion sites are as shown below based on the bregma (B: 0 mm, ML coordinate: 0 mm (median)).

B: the distance in the anterior-posterior axial direction based on the bregma (a plus value indicates the distance in the front)
L: the distance to the left based on the ML coordinate
D: the depth in the brain from the dura mater in the vertical downward direction

An endothelin-1 (Peptide Institute, Inc. code 4198-v) solution was infused to the four individuals of male cynomolgus monkey. Specifically, in the skull of each cynomolgus monkey, holes with a diameter of around 1 mm were made with a drill at locations (i) B: 2.0 mm, L: 8.0 mm, (ii) B: 5.0 mm, L: 8.0 mm, (iii) B: 9.0 mm, L: 5.0 mm and (iv) B: 9.0 mm, L: 12.0 mm (four locations in total) based on the bregma for the infusion sites. A micro syringe (HAMILTON) connected to a microinjector (Narishige Group) was inserted from each hole and placed in such a manner that the needle tip was at the depth (D) 19.0 mm ((i) and (ii) above), the depth (D) 13.0 mm ((iii) above) or the depth (D) 15.0 mm ((iv) above) from the dura mater. Endothelin-1 was infused to the four sites in total corresponding to the locations for each individual using the micro syringes. Endothelin-1 was infused at a concentration of 2.0 μg/μL in an amount of 30 μL/site at an infusion rate of 1.5 μL/min. After the completion of the infusion, the syringes were left still for about 20 minutes to prevent any liquid leakage. The infusion sites (i) to (iv) above target (i) the ventral lateral thalamic nucleus and the ventral posterior thalamic nucleus, (ii) the globus pallidus, (iii) the caudate nucleus and (iv) the putamen, respectively. By infusing endothelin to the infusion sites as described above, damage was caused to the internal capsule in addition to the targeted sites. In this manner, a cerebral infarction model having basal ganglia disorder, thalamus disorder and internal capsule disorder was produced.

(Administration of ND1 Nucleic Acid Lipid Nanoparticles to Model)

On day 21 after the endothelin-1 infusion treatment (chronic phase), the four individuals of the cerebral infarction model produced by the above method were divided into two groups (a control group with n=1 and an ND1 nucleic acid lipid nanoparticle group with n=3) in such a manner that there was no difference between the groups regarding the mRS for monkeys (see the table below). Here, Ex1-L1c-eGFP mRNA prepared in Example 10 below was administered to the control group (one individual), and Ex1-L1-ND1-1 prepared in Example 10 below was administered to the ND1 nucleic acid lipid nanoparticle group (three individuals).

For medical examination of human stroke, the mRS (for human clinical uses) described in Table 4 is widely used as an indicator of disability. The mRS (for monkeys) is an indicator in which the mRS (for human clinical uses) is modified for monkeys. As an indicator used for scoring monkeys' motor dysfunction, the Non-Human Primate Stroke Scale (NHPSS), which is an evaluation scale obtained by adopting the National Institutes of Health Stroke Scale (NIHSS), which is an evaluation scale for stroke neurological severity of humans, to nonhuman primate models, is known. Because the mRS (for monkeys) can evaluate the durability of motor dysfunction like the NHPSS, the mRS can be used for evaluating the durability of motor dysfunction of this model.

[Table 4]

| Score | mRS (for human clinical practice) | mRS (for monkeys) |
|---|---|---|
| 0 | No symptoms at all | No symptoms at all |

(continued)

| Score | mRS (for human clinical practice) | mRS (for monkeys) |
|---|---|---|
| 1 | No significant disability: despite symptoms, able to carry out all usual duties and activities | Mild paralysis[1] of a forelimb or hindlimb on impaired side |
| 2 | Slight disability: unable to perform all previous activities but able to look after own affairs without assistance | Slight decline in locomotor activity[3], or mild paralysis of a forelimb and hindlimb on impaired side |
| 3 | Moderate disability: requiring some help but able to walk without assistance | (i) Decline in locomotor activity[4], or (ii) seated posture (including incomplete standing-up motion), or (iii) paralysis[2] of a forelimb and hindlimb on impaired side. No decreased level of consciousness[5] |
| 4 | Moderately severe disability: unable to walk without assistance and unable to attend to own bodily needs without assistance | (i) Decline in locomotor activity and (ii) seated posture/recumbent posture[6] (including incomplete standing-up motion), and (iii) paralysis of a forelimb and hindlimb on impaired side or a slightly decreased level of consciousness[7] |
| 5 | Severe disability: bedridden, incontinent and requiring constant nursing care and attention | Recumbent posture (including incomplete standing-up motion), and paralysis of a forelimb and hindlimb on impaired side, and a decreased level of consciousness[8] |
| 6 | Death | Death |

[1]Mild paralysis: no significant paralysis is found, but a slight sign of paralysis is found.
[2]Paralysis: significant paralysis is found.
[3]Slight decline in locomotor activity: no significant decline in locomotor activity is found, but a slight sign of decline in locomotor activity is found.
[4]Decline in locomotor activity: significant decline in locomotor activity is found.
[5]Level of consciousness (monkeys): determined on the level of response to human.
[6]Seated posture/recumbent posture: at least one of seated posture and recumbent posture.
[7]Slightly decreased level of consciousness: no significantly decreased level of consciousness is found, but a slight sign of a decreased level of consciousness is found.
[8]Decreased level of consciousness: a significantly decreased level of consciousness is found.

The cerebral infarction model individuals of the groups were subjected to induction anesthesia through i.m. of 15 to 50 mg/0.3 to 1.0 mL/head of ketamine hydrochloride and maintenance anesthesia through isoflurane inhalation. Under continuous anesthesia, the hair on the head was shaved with clippers, and then each model individual was fixed with a brain stereotaxic apparatus. Next, the operation part was disinfected, and the skin of the head was cut open to expose the bregma with consideration for bleeding.

For each of the cerebral infarction model individuals of the groups, a micro syringe was placed in each of the four holes in total through which endothelin-1 was infused when the cerebral infarction model was produced in Test Example 5 in such a manner that the needle tip was at the same depth (D) as that in Test Example 5. Ex1-L1-ND1-1 was administered to three individuals, and Ex1-L1c-eGFP mRNA was administered to one individual. The nucleic acid lipid nanoparticles were prepared at 0.3 mg/mL with PBS and administered at 30 μL/site at an administration rate of 1.5 μL/min. After the completion of the administration, the syringes were left still for about 20 minutes.

(Observation of Motor Function)

The individuals of the ND1 nucleic acid lipid nanoparticle group and the control group in (Administration of Nucleic Acid Lipid Nanoparticles to Model) above were observed before the endothelin-1 infusion treatment (day 0) and on days 1, 7, 14, 21, 22, 28, 42, 56, 70 and 84 after the endothelin-1 infusion treatment based on the mRS (Table 4). Here, as described above, the behaviors were evaluated every two weeks on and after day 28 after the endothelin-1 infusion treatment, and as the evaluation of the behaviors on day 56 after the endothelin-1 infusion treatment (day 35 after the administration of the nucleic acid lipid nanoparticles), the individuals were evaluated on day 57, 58 or 59 after the

endothelin-1 infusion treatment (day 36, 37 or 38 after the administration of the nucleic acid lipid nanoparticles). As the evaluation of the behavior of the control group on day 56 after the endothelin-1 infusion treatment (day 35 after the administration of the nucleic acid lipid nanoparticles), the one individual of the control group was evaluated on day 58 after the endothelin-1 infusion treatment (day 37 after the administration of the nucleic acid lipid nanoparticles). The results were used in this Example as the results on day 56 after the endothelin-1 infusion (day 35 after the administration of the nucleic acid lipid nanoparticles).

As a result, in both of the ND1 nucleic acid lipid nanoparticle group and the control group, there was no difference in the motor function on day 1 after the administration, and similar motor dysfunction to that before the administration of the nucleic acid lipid nanoparticles was observed. In the control group, however, the motor function was not improved even on day 84 after the endothelin-1 infusion (day 63 after the administration of the nucleic acid lipid nanoparticles), while in the ND1 nucleic acid lipid nanoparticle group, the motor function was recovered on and after day 56 after the endothelin-1 infusion (day 35 after the administration of the nucleic acid lipid nanoparticles), and significant recovery of the motor function was observed on day 84 after the endothelin-1 infusion (day 63 after the administration of the nucleic acid lipid nanoparticles) (Fig. 1). This shows that administration of the ND1 nucleic acid lipid nanoparticles can recover motor dysfunction following brain damage.

Moreover, because it was suggested in Test Example 4 that Ex1-L6-ND1-2 also converted astrocytes into neurons like Ex1-L1-ND1-1, it is expected that a similar effect of ND1 will be exhibited like Ex1-L1-ND1-1 also when Ex1-L6-ND1-2 is administered.

From the above results, it was found that administration of ND1 nucleic acid lipid nanoparticles exhibits a therapeutic effect also on cerebral infarction (especially, perforator infarction, cerebral infarction having brain damage in the penetrating branch territory; subacute-phase to chronic-phase cerebral infarction; or cerebral infarction having motor dysfunction with high severity).

From the results of the above Test Examples, using the compound of the formula (I) or a salt thereof as a cationic lipid, lipid nanoparticles which are suitable for a pharmaceutical composition for preventing and/or treating an astrocyte-related disease can be produced. Examples

The production method of the compound of the formula (I) or a salt thereof will be explained in further detail below based on Examples. In this regard, the invention is not limited to the compounds described in the following Examples. The production methods of raw material compounds are also shown in Production Examples. The production method of the compound of the formula (I) or a salt thereof is not limited only to the production methods of specific Examples described below, and the compound of the formula (I) or a salt thereof can also be produced by a combination of the production methods or a method that is obvious to one skilled in the art.

The abbreviations below are sometimes used in this specification, Examples, Production Examples and the tables.

Nucleic acid lipid nanoparticles: lipid nanoparticles encapsulating a nucleic acid, DCM: dichloromethane, DMAP: N,N-dimethyl-4-aminopyridine (also known as 4-(dimethylamino)pyridine), DIPEA: N,N-diisopropylethylamine, DMF: N,N-dimethylformamide, DMG-PEG2000:1,2-Dimyristoyl-rac-glycero-3-methoxypolyethylene glycol PEG(also known as 1,2-Dimyristoyl-rac-glycero-3- methoxyPEG or DMG-PEG), DMSO: dimethyl sulfoxide, TEA: triethylamine, THF: tetrahydrofuran, DSPC: 1,2-distearoyl-sn-glycero-3-phosphocholine, and TE buffer: 10 mM Tris/1 mM EDTA buffer (pH 7.5 to 8.0). NUM: Example number or Production Example number, PEx: Production Example compound, and Ex: Example compound ("Ex number": Example compound). "Ex number-L number-*** mRNA": lipid nanoparticles encapsulating "*** mRNA", containing "Ex number" and having the lipid composition and the composition ratio of "L number". "Ex number-L number" (not having "-*** mRNA" at the end): lipid nanoparticles encapsulating mRNA that expresses a fluorescent protein, containing "Ex number" and having the lipid composition and the composition ratio of "L number". (For example, Ex1-L1 in the tables indicates Example lipid nanoparticles encapsulating mRNA that expresses a fluorescent protein, containing the Example compound Ex1 and having the lipid composition and the composition ratio of Ex1-L1.) REF: Example or Production Example used as reference. (For example, Ex2 in REF of Ex4 in the table below shows that the Example compound 4 (Ex4) can be produced by the same method as that in the Example texts of the Example compound 2 (Ex2).) STR: chemical structural formula, DAT: physiochemical data, ESI+: m/z value in ESI-MS+, and NMR: $\delta$ value (ppm) of $^1$H-NMR (500 MHz) using $CDCl_3$ as the measurement solvent. NMR signal shows a typical signal. s: singlet, d: doublet, t: triplet, q: quartet, quin: quintet, td: triple doublet, m: multiplet, br: broad, brd: broad doublet, and brt: broad triplet. In this specification, a compound is sometimes named using naming software, such as ACD/Name (registered trademark, Advanced Chemistry Development, Inc.). For the purpose of convenience, the concentration mol/L is shown as M. For example, 1 M aqueous sodium hydroxide solution means an aqueous sodium hydroxide solution of 1 mol/L.

Production Example 1

Under nitrogen atmosphere, DMAP (61.9 g) and 1-(3-dimethylaminopropyl)-3-ethylcarbodiimide hydrochloride

(97.2 g) were added to a mixture of 8-bromooctanoic acid (75.5 g) and 9-heptadecanol (86.8 g) in DMF (755 mL) under ice cooling. Then, the mixture was stirred at room temperature for 28 hours. The reaction mixture was added to ice water and subjected to extraction with toluene. The aqueous layer was subjected to extraction with toluene, and the combined organic layer was washed with aqueous saturated sodium chloride solution. The wash solution was subjected to extraction with toluene and ethyl acetate, and the combined organic layer was dried over anhydrous magnesium sulfate. The organic layer was concentrated under reduced pressure. Then, hexane was added, and the mixture was concentrated again under reduced pressure. The obtained residue was purified by silica gel column chromatography (hexane/ethyl acetate), and thus heptadecan-9-yl 8-chlorooctanoate (80.3 g) was obtained.

Example 1

Under nitrogen atmosphere, potassium carbonate (1.7 g) and potassium iodide (51 mg) were added to a mixture of 2-(4-amino-1-piperidinyl)ethanol dihydrochloride (672 mg), heptadecan-9-yl 8-chlorooctanoate (3.0 g) and N,N-dimethylacetamide (6.7 mL). After stirring at an internal temperature of 100°C for 24 hours, the reaction mixture was allowed to cool at room temperature, and the insoluble matter was removed by filtration and washed with toluene. Water was added to the filtrate, and the organic layer was extracted. The aqueous layer was subjected to extraction with toluene and ethyl acetate, and the combined organic layer was washed with aqueous saturated sodium chloride solution. The aqueous layer was subjected to extraction with ethyl acetate, and the combined organic layer was dried over anhydrous sodium sulfate. The organic layer was concentrated under reduced pressure, and the obtained residue was purified by silica gel column chromatography (hexane/ethyl acetate) and silica gel column chromatography (chloroform/methanol). The obtained crude purified product was purified by silica gel column chromatography (basic silica gel, hexane/ethyl acetate), and thus di(heptadecan-9-yl) 8,8'-{[1-(2-hydroxyethyl)piperidin-4-yl]azanediyl}di(octanoate) (540 mg) was obtained.

Production Example 2

Under nitrogen atmosphere, DMAP (250 g) and 1-(3-dimethylaminopropyl)-3-ethylcarbodiimide hydrochloride (392 g) were sequentially added to a mixture of 7-tridecanol (273 g), 9-bromononanoic acid (324 g) and DMF (3.24 L) under ice cooling. Then, the mixture was stirred at room temperature for three days. The reaction mixture was added to a mixture solution of aqueous saturated sodium chloride solution, aqueous saturated sodium hydrogen carbonate solution and ice, and the mixture was subjected to extraction with (hexane/ethyl acetate)=(1:1). The aqueous layer was subjected to extraction with (hexane/ethyl acetate)=(1:1), and the combined organic layer was washed with aqueous saturated sodium chloride solution. The organic layer was dried over anhydrous magnesium sulfate and then concentrated under reduced pressure. The obtained residue was purified by silica gel chromatography (hexane/ethyl acetate), and thus tridecan-7-yl 9-chlorononanoate (293 g) was obtained.

Example 2

Under argon atmosphere, a mixture of 2-(4-amino-1-piperidinyl)ethanol (175 mg), tridecan-7-yl 9-chlorononanoate (1.17 g), potassium iodide (40 mg), potassium carbonate (402 mg), DIPEA (415 μL), cyclopentyl methyl ether (5 mL) and acetonitrile (5 mL) was stirred under irradiation with microwaves at 160°C for 12 hours. The insoluble matter in the reaction mixture was removed by filtration, and the filtrate was concentrated under reduced pressure. The obtained residue was crudely purified by silica gel chromatography (basic silica gel, hexane/ethyl acetate), and then the mixture fraction was purified by silica gel chromatography (chloroform/methanol). Thus, di(tridecan-7-yl) 9,9'-{[1-(2-hydroxyethyl)piperidin-4-yl]azanediyl}di(nonanoate) (141.8 mg) was obtained.

Production Example 3

A mixture of 2-hexyldecanoic acid (10 g), 1-(3-dimethylaminopropyl)-3-ethylcarbodiimide hydrochloride (8.3 g), 9-bromo-1-nonanol (16.5 g), DMAP (0.96 g) and dichloromethane (200 mL) was stirred at room temperature overnight. Dichloromethane was added to the reaction mixture, and aqueous saturated sodium hydrogen carbonate solution was added to separate the solutions. The organic layer was washed with aqueous saturated sodium chloride solution, then dried over anhydrous magnesium sulfate and concentrated under reduced pressure. The obtained residue was purified by silica gel chromatography (hexane/ethyl acetate), and thus 9-bromononyl 2-hexyldecanoate (16.8 g) was obtained.

Example 3

Under argon atmosphere, a mixture of 2-(4-amino-1-piperidinyl)ethanol dihydrochloride (299 mg), 9-bromononyl 2-hexyldecanoate (1.41 g), potassium iodide (46 mg), potassium carbonate (609 mg), cyclopentyl methyl ether (0.7 mL) and acetonitrile (0.7 mL) was stirred under irradiation with microwaves at 120°C for six hours and further stirred under irradiation with microwaves at 100°C for 10 hours. The reaction mixture was filtered through celite, and the filtrate was concentrated under reduced pressure. The obtained residue was purified by silica gel chromatography (basic silica gel, hexane/ethyl acetate), and thus {[1-(2-hydroxyethyl)piperidin-4-yl]azanediyl}di(nonane-9,1-diyl) bis(2-hexyldecanoate) (348 mg) was obtained.

[Table 5]

| NUM | STR |
|---|---|
| PEx1 | 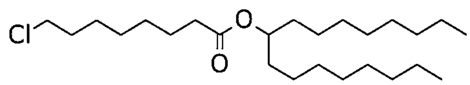 |
| Ex1 | 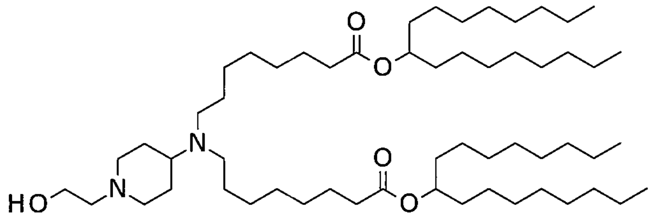 |
| PEx2 | 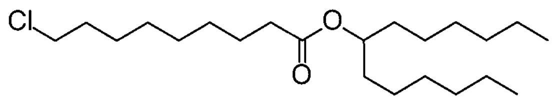 |
| Ex2 | 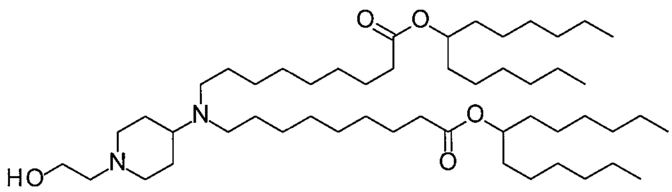 |
| PEx3 | 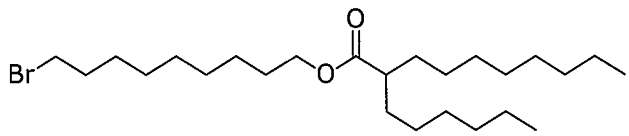 |
| Ex3 | 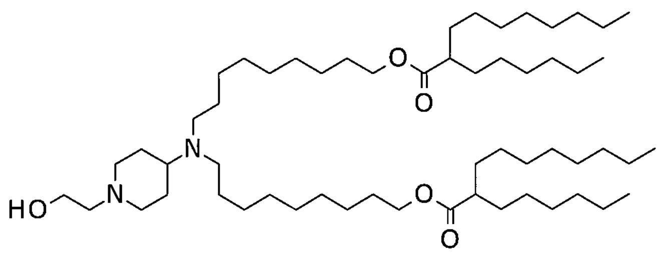 |

[Table 6]

| NUM | STR |
|---|---|
| PEx4 | 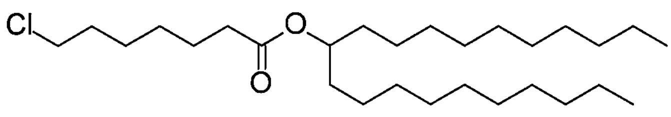 |

(continued)

| NUM | STR |
|---|---|
| Ex4 | 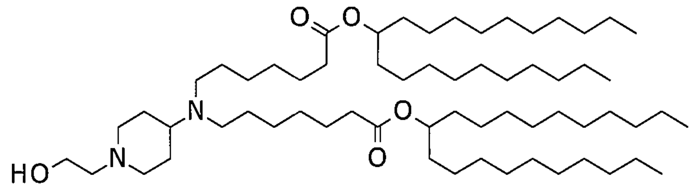 |
| PEx5 | 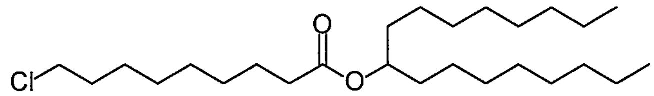 |
| Ex5 | 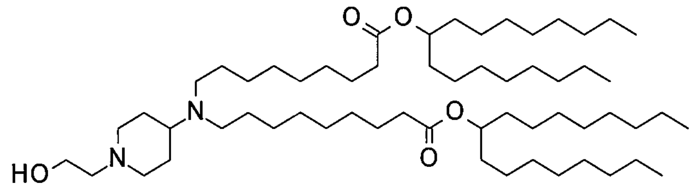 |

[Table 7]

| Num | REF | DAT |
|---|---|---|
| PEx1 | PEx1 | ESI+:440 |
| Ex1 | Ex1 | ESI+:906<br>NMR:0.88(t,J=7.00Hz,12H),1.23-1.42(m,66H),1.45-1.74(m,14H),2.04 (td, J=11.80,1.98Hz,2H),2.27(t,J=7.57Hz,4H),2.36-2.52(m,7H),2.94 (brd ,J= 11.47Hz, 2H), 3.58(t, J=5.43Hz, 2H) ,4.86(quin,J=6.23Hz,2H) |
| PEx2 | PEx2 | ESI+:397 |
| Ex2 | Ex2 | ESI+: 822<br>NMR:0.88(t,J=6.98Hz,12H),1.20-1.44(m,54H),1.46-1.75(m,14H),2.00-2.09 (m,2H),2.27(t,J=7.50Hz,4H),2.36-2.55(m,7H),2.95(brd,J=11.47Hz, 2H),3.59(t, J=5.43Hz,2H),4.87(quin,J=6.27Hz,2H) |
| PEx3 | PEx3 | ESI+:463 |
| Ex3 | Ex3 | ESI+:906<br>NMR:0.88(td,J=7.00,1.30Hz,12H),1.15-1.79(m,80H),2.04(td,J=11.78, 1.99Hz, 2H),2.31(tt,J=8.99,5.24Hz,2H),2.38-2.44(m,4H),2.45-2.53(m, 3H) ,2.95(brd, J=11.47Hz,2H),3.58(t,J=5.35Hz,2H),4.06(brd,J=6.68Hz, 4H) |
| PEx4 | PEx1 | ESI+:481 |
| Ex4 | Ex2 | ESI+:990<br>NMR:0.89(t,J=6.90Hz,12H),1.19-1.75(m,92H),2.00-2.08(m,2H),2.28(t, J= 7.57Hz,4H),2.36-2.53(m,7H),2.96(brd,J= 11.63Hz, 2H),3.59 (t,J=5.43Hz,2H),4.87(quin,J=6.23Hz,2H) |
| PEx5 | PEx1 | ESI+:453 |
| Ex5 | Ex2 | ESI+:934<br>NMR:0.89(t,J=6.93Hz,12H),1.17-1.80(m,84H),2.05(brt,J=11.09Hz,2H), 2.28 (t,J=7.57Hz,4H),2.37-2.54(m,7H),2.96(brd,J= 11.47Hz, 2H), 3.59(t,J=5.35Hz,2H),4.87(quin,J=6.27Hz,2H) |

Example 10: Production of Nucleic Acid Lipid Nanoparticles

(Raw Materials of Nucleic Acid Lipid Nanoparticles)

In the preparation of nucleic acid lipid nanoparticles below, the following raw materials were used. That is: DSPC was manufactured by NOF corporation (product name: COATSOME (registered trademark) MC-8080); the cholesterol was manufactured by Nippon Fine Chemical Co., Ltd. (product name: Cholesterol HP); DMG-PEG2000 was manufactured by NOF corporation (SUNBRIGHT (registered trademark) GM-020); and the mRNA was manufactured by TriLink BioTechnologies (product name: CleanCap (registered trademark) FLuc mRNA or CleanCap (registered trademark) eGFP mRNA).

(Preparation of Nucleic Acid Lipid Nanoparticles)

A cationic lipid obtained in Examples 1 to 5, DSPC, the cholesterol and DMG-PEG2000 were dissolved in ethanol at any lipid composition ratio and any N/P ratio, and an oil phase was obtained. Using 6.25 mM acetate buffer (pH 5), 10 mM citrate buffer (pH 4) or 10 mM citrate buffer (pH 6) containing mRNA as an aqueous phase, the aqueous phase and the oil phase were mixed at a volume ratio of aqueous phase:oil phase=3:1 with a microfluidic device (NanoAssemblr, manufactured by Precision NanoSystems), and the mixture solution was diluted with phosphate-buffered saline (PBS). Thus, a dispersion of nucleic acid lipid nanoparticles was obtained. Ethanol was removed through dialysis or ultrafiltration of the dispersion. Subsequently, the dispersion was concentrated through ultrafiltration to prepare to any concentration.

In this regard, Ex1-L0 to Ex5-L0 and Ex1-L1 to Ex1-L17 were prepared using acetate buffer or citrate buffer. Specifically, for Ex1-L0 to Ex3-L0, the nucleic acid lipid nanoparticles were prepared using 6.25 mM acetate buffer (pH 5) containing FLuc mRNA as the aqueous phase by preparing the oil phase at cationic lipid | DSPC | cholesterol| DMG-PEG2000=50 | 10 | 38.5 | 1.5 (mol%) and N/P ratio=6. For Ex4-L0 to Ex5-L0, the nucleic acid lipid nanoparticles were prepared using 10 mM citrate buffer (pH 4) containing FLuc mRNA as the aqueous phase by preparing the oil phase at cationic lipid | DSPC | cholesterol| DMG-PEG2000=50 | 10 | 38.5 | 1.5 (mol%) and N/P ratio=6. For Ex1-L01, the nucleic acid lipid nanoparticles were prepared using 10 mM citrate buffer (pH 6) containing FLuc mRNA as the aqueous phase by preparing the oil phase at cationic lipid | DSPC | cholesterol | DMG-PEG2000=50 | 10 | 38.5 | 1.5 (mol%) and N/P ratio=6. For Ex1-L1 to Ex1-L17, the nucleic acid lipid nanoparticles were prepared using 10 mM citrate buffer (pH 6) containing eGFP mRNA as the aqueous phase by preparing the oil phase at the lipid composition ratio and the N/P ratio in the table below. Ex1-L1 to Ex1-L17 shown in NUM in the table below indicate Ex1-L1-eGFP mRNA to Ex1-L17-eGFP mRNA, respectively.

[Table 8]

| NUM | Lipid Composition (mol%) Ex1 \| DSPC \| Cholesterol \| DMG-PEG2000 | N/P Ratio |
|---|---|---|
| Ex1-L1 | 50 \| 10 \| 38.5 \| 1.5 | 6 |
| Ex1-L2 | 20 \| 16 \| 62.5 \| 1.5 | 6 |
| Ex1-L3 | 25 \| 15 \|58.5 \| 1.5 | 6 |
| Ex1-L4 | 30 \| 14 \| 54.5 \| 1.5 | 6 |
| Ex1-L5 | 35 \| 13 \| 50.5 \|1.5 | 6 |
| Ex1-L6 | 40 \| 12 \| 46.5 \| 1.5 | 6 |
| Ex1-L7 | 60 \| 8 \| 30.5 11.5 | 6 |
| Ex1-L8 | 70 \| 6 \| 22.5 \|1.5 | 6 |
| Ex1-L9 | 40 \| 0 \| 58.5 \| 1.5 | 6 |
| Ex1-L10 | 40 \| 5 \| 53.5 \| 1.5 | 6 |
| Ex1-L11 | 40 \|12 \| 47.5 \| 0.5 | 6 |
| Ex1-L12 | 40 \| 12 \| 47 \| 1 | 6 |
| Ex1-L13 | 40 \| 12 \| 46 \| 2 | 6 |
| Ex1-L14 | 40 \|12 \| 45.5 \| 2.5 | 6 |
| Ex1-L15 | 40 \| 12 \| 45 \| 3 | 6 |
| Ex1-L16 | 50 \| 10 \| 38.5 \| 11.5 | 12 |

(continued)

| NUM | Lipid Composition (mol%) Ex1 \| DSPC \| Cholesterol \| DMG-PEG2000 | N/P Ratio |
|---|---|---|
| Ex1-L17 | 40 \| 12 \| 46.5 \| 1.5 | 12 |

Ex1-L1-eGFP mRNA was pharmacologically evaluated in Test Example 2, and the percentages of the components with fluorescent intensities of 0.1 or more and 1.0 or more based on the fluorescent intensity thereof regarded as a basis of 1 were calculated. As a result, the composition ratio of Ex1 was 20.0 to 70.0 mol% or 35.0 to 70.0 mol% based on the total amount of the lipid nanoparticles. The composition ratio of the phospholipid was 0 to 16.0 mol% or 0 to 13.0 mol% based on the total amount of the lipid nanoparticles. The composition ratio of sterol was 22.5 to 62.5 mol% or 22.5 to 58.5 mol% based on the total amount of the lipid nanoparticles. The composition ratio of the PEGylated lipid was 0.5 to 3.0 mol% or 1.0 to 2.5 mol% based on the total amount of the lipid nanoparticles.

(Raw Materials of ND1 Nucleic Acid Lipid Nanoparticles)

Two types of ND1 mRNA encoding human ND1 ((i) mRNA which had a base sequence containing a 5' UTR and a 3' UTR provided from TriLink BioTechnologies, CDS of human ND1 gene (SEQ ID NO: 1) and a poly(A) sequence of 120 bases and in which the 5' cap structure was Cap-1 (called "ND1-1" below) and (ii) mRNA which had a base sequence (SEQ ID NO: 5) in that all the uridines were substituted with N1-methylpseudouridines in a base sequence (SEQ ID NO: 4) containing a 5' UTR derived from human $\alpha$-globin gene, a 3' UTR derived from human $\alpha$-globin gene, CDS of human ND1 gene (SEQ ID NO: 3) and a poly(A) sequence of 79 bases and in which the 5' cap structure was Cap-1 (called "ND1-2" below)) were produced (outsourced to TriLink BioTechnologies). In this regard, in the base sequence of SEQ ID NO: 4 or SEQ ID NO: 5, the nucleotides at positions 1 to 3 correspond to AGG, positions 4 to 43 correspond to the 5' UTR, positions 44 to 1114 correspond to CDS of human NeuroD1 gene (SEQ ID NO: 3), positions 1115 to 1120 correspond to two successive stop codons, positions 1121 to 1231 correspond to the 3' UTR, and positions 1232 to 1310 correspond to the poly(A) sequence. As the control, eGFP mRNA (TriLink BioTechnologies, L-7601) was used.

(Preparation of ND1 Nucleic Acid Lipid Nanoparticles)

Ex1, which is a cationic lipid, DSPC, the cholesterol and DMG-PEG2000 were dissolved in ethanol at N/P ratio=6, and an oil phase was obtained. Using 10 mM citrate buffer (pH 4 or 6) containing mRNA as an aqueous phase, the aqueous phase and the oil phase were mixed at a volume ratio of aqueous phase:oil phase=3:1 with a microfluidic device (NanoAssemblr, Precision NanoSystems), and the mixture solution was diluted with phosphate-buffered saline (PBS). Thus, a dispersion of nucleic acid lipid nanoparticles was obtained. Ethanol was removed through dialysis or ultrafiltration of the dispersion. Subsequently, the dispersion was concentrated through ultrafiltration to prepare to any concentration.

The lipid nanoparticles Ex1-L1, which were obtained using citrate buffer (pH 4) as the aqueous phase and which encapsulated ND1-1, are called Ex1-L1-ND1-1, and the lipid nanoparticles Ex1-L6, which were obtained using citrate buffer (pH 6) as the aqueous phase and which encapsulated ND1-2, are called Ex1-L6-ND1-2. Similarly, the lipid nanoparticles which were obtained using citrate buffer (pH 4) as the aqueous phase and which encapsulated eGFP mRNA and had the lipid composition shown in Ex1-L1 are called Ex1-L1c-eGFP mRNA.

(Measurement of Particle Size)

The particle sizes of the nucleic acid lipid nanoparticles were measured using the nucleic acid lipid nanoparticle dispersions using a particle size analyzer (Zetasizer Nano ZSP, manufactured by Malvern Panalytical).

(Evaluation of Encapsulation Efficiency of mRNA)

Regarding the nucleic acid lipid nanoparticles, the encapsulation efficiencies of mRNA in the nucleic acid lipid nanoparticle dispersions which were diluted to an mRNA concentration of around 400 to 3500 ng/mL were measured. Specifically, the nucleic acid lipid nanoparticles of each of Ex1-L0 to Ex5-L0 and Ex1-L1 to Ex1-L17 obtained in accordance with "(Preparation of Nucleic Acid Lipid Nanoparticles)" above were diluted with TE buffer (10 mM Tris/1 mM EDTA, pH 7.5 to 8.0), and the mRNA concentration (A) measured using Quant-iT RiboGreen RNA Reagent (manufactured by Thermo Fisher Scientific) was regarded as the mRNA existing in the external liquid of the nucleic acid lipid nanoparticles. Moreover, the mRNA concentration (B) measured after diluting the nucleic acid lipid nanoparticles with 2% Triton X-100 was regarded as the total mRNA concentration in the composition. Subsequently, the encapsulation efficiency of mRNA was calculated by the following equation (F1).

$$\text{Encapsulation efficiency (\%)} = 100 - (A/B) \times 100 \ldots \text{(F1)}$$

The particle sizes of the nucleic acid lipid nanoparticles and the encapsulation efficiencies of mRNA are shown in the tables below. Ex1-L0 to Ex5-L0 shown in NUM in the table below indicate Ex1-LO-FLuc mRNA to Ex5-LO-FLuc mRNA, respectively, and Ex1-L1 to Ex1-L17 indicate Ex1-L1-eGFP mRNA to Ex1-L17-eGFP mRNA, respectively.

[Table 9]

| NUM | Particle Size (nm) | Encapsulation Efficiency (%) |
|---|---|---|
| Ex1-L0 | 116.0 | 95.6 |
| Ex2-L0 | 98.5 | 99.0 |
| Ex3-L0 | 111.5 | 97.1 |
| Ex4-L0 | 96.1 | 98.9 |
| Ex5-L0 | 91.1 | 98.8 |

[Table 10]

| NUM | Particle Size (nm) | Encapsulation Efficiency (%) |
|---|---|---|
| Ex1-L1 | 108.9 | 96.0 |
| Ex1-L2 | 64.0 | 93.8 |
| Ex1-L3 | 66.6 | 95.5 |
| Ex1-L4 | 68.0 | 94.8 |
| Ex1-L5 | 76.5 | 96.4 |
| Ex1-L6 | 84.7 | 95.9 |
| Ex1-L7 | 141.9 | 95.3 |
| Ex1-L8 | 175.3 | 86.9 |
| Ex1-L9 | 111.6 | 94.8 |
| Ex1-L10 | 91.1 | 94.9 |
| Ex1-L11 | 109.5 | 94.9 |
| Ex1-L12 | 96.5 | 96.0 |
| Ex1-L13 | 78.3 | 96.1 |
| Ex1-L14 | 73.2 | 96.0 |
| Ex1-L15 | 65.5 | 94.9 |
| Ex1-L16 | 109.3 | 95.6 |
| Ex1-L17 | 94.1 | 94.2 |

Industrial Applicability

Using the compound of the formula (I) or a salt thereof, lipid nanoparticles can be formed, and such lipid nanoparticles are introduced into cells, such as astrocytes. Using such lipid nanoparticles produced using the compound of the formula (I) or a salt thereof, a pharmaceutical composition can also be produced. Lipid nanoparticles and a pharmaceutical composition using mRNA or the like are expected to be useful for preventing and/or treating an astrocyte-related disease.

[Sequence Listing Free Text]

SEQ ID NO: 1: Human NeuroD1 gene (CDS)

SEQ ID NO: 2: Human NeuroD 1 protein
SEQ ID NO: 3: Human NeuroD1 gene (CDS)
SEQ ID NO: 4: Human NeuroD 1 mRNA sequence (ND 1-2)
SEQ ID NO: 5: Human NeuroD1 mRNA sequence containing modified nucleotides (ND 1-2)

Sequence Listing

# Sequence Listing

| | | |
|---|---|---|
| **1** | **Sequence Listing Information** | |
| 1-1 | File Name | A23027A00.xml |
| 1-2 | DTD Version | V1_3 |
| 1-3 | Software Name | WIPO Sequence |
| 1-4 | Software Version | 2.3.0 |
| 1-5 | Production Date | 2023-12-12 |
| 1-6 | Original free text language code | |
| 1-7 | Non English free text language code | |
| **2** | **General Information** | |
| 2-1 | Current application: IP Office | |
| 2-2 | Current application: Application number | |
| 2-3 | Current application: Filing date | |
| 2-4 | Current application: Applicant file reference | A23027A00 |
| 2-5 | Earliest priority application: IP Office | JP |
| 2-6 | Earliest priority application: Application number | 2022-211994 |
| 2-7 | Earliest priority application: Filing date | 2022-12-28 |
| 2-8en | Applicant name | Astellas Pharma Inc. |
| 2-8 | Applicant name: Name Latin | |
| 2-9 | Inventor name | |
| 2-9 | Inventor name: Name Latin | |
| 2-10en | Invention title | 4-AMINOPIPERIDINE COMPOUND, LIPID NANOPARTICLES THEREOF AND PHARMACEUTICAL COMPOSITION CONTAINING THE SAME |
| 2-10ja | Invention title | ４－アミノピペリジン化合物、その脂質ナノ粒子、及び、それを含む医薬組成物 |
| 2-11 | Sequence Total Quantity | 5 |
| **3-1** | **Sequences** | |
| 3-1-1 | Sequence Number [ID] | 1 |
| 3-1-2 | Molecule Type | DNA |
| 3-1-3 | Length | 1071 |
| 3-1-4-1 | Features Location/Qualifiers | **source** 1..1071<br>mol_type= other DNA<br>organism= Homo sapiens |
| | NonEnglishQualifier Value | |
| 3-1-4-2 | Features Location/Qualifiers | **misc_feature** 1..1071<br>note= Human NeuroD1 Gene (CDS) |
| | NonEnglishQualifier Value | |
| 3-1-5 | **Residues**<br>atgaccaaat cgtacagcga gagtgggctg atgggcgagc ctcagcccca aggtcctcca 60<br>agctggacag acgagtgtct cagttctcag gacgaggagc acgaggcaga caagaaggag 120<br>gacgacctcg aaaccatgaa cgcagaggag gactcactga ggaacggggg agaggaggag 180 | |

| | | |
|---|---|---|
| | gacgaagatg aggacctgga agaggaggaa gaagaggaag aggaggatga cgatcaaaag 240<br>cccaagagac gcggccccaa aaagaagaag atgactaagg ctcgcctgga gcgttttaaa 300<br>ttgagacgca tgaaggctaa cgcccgggag cggaaccgca tgcacggact gaacgcggcg 360<br>ctagacaacc tgcgcaaggt ggtgccttgc tattctaaga cgcagaagct gtccaaaatc 420<br>gagactctgc gcttggccaa gaactacatc tgggctctgt cggagatcct gcgctcaggc 480<br>aaaagcccag acctggtctc cttcgttcag acgctttgca agggcttatc ccaacccacc 540<br>accaacctgg ttgcgggctg cctgcaactc aatcctcgga cttttctgcc tgagcagaac 600<br>caggacatgc ccccccacct gccgacggcc agcgcttcct tccctgtaca cccctactcc 660<br>taccagtcgc ctgggctgcc cagtccgcct tacggtacca tggacagctc ccatgtcttc 720<br>cacgttaagc ctccgccgca cgcctacagc gcagcgctgg agcccttctt tgaaagccct 780<br>ctgactgatt gcaccagccc ttcctttgat ggacccctca gcccgccgct cagcatcaat 840<br>ggcaacttct ctttcaaaca cgaaccgtcc gccgagtttg agaaaaatta tgcctttacc 900<br>atgcactatc ctgcagcgac actggcaggg gcccaaagcc acggatcaat cttctcaggc 960<br>accgctgccc ctcgctgcga gatccccata gacaatatta tgtccttcga tagccattca 1020<br>catcatgagc gagtcatgag tgcccagctc aatgccatat ttcatgatta g 1071 | |
| **3-2** | **Sequences** | |
| 3-2-1 | Sequence Number [ID] | 2 |
| 3-2-2 | Molecule Type | AA |
| 3-2-3 | Length | 356 |
| 3-2-4-1 | Features Location/Qualifiers | **source** 1..356<br>mol_type= protein<br>note= Human NeuroD1 Protein<br>organism= Homo sapiens |
| | NonEnglishQualifier Value | |
| 3-2-5 | **Residues**<br>MTKSYSESGL MGEPQPQGPP SWTDECLSSQ DEEHEADKKE DDLETMNAEE DSLRNGGEEE 60<br>DEDEDLEEEE EEEEEDDDQK PKRRGPKKKK MTKARLERFK LRRMKANARE RNRMHGLNAA 120<br>LDNLRKVVPC YSKTQKLSKI ETLRLAKNYI WALSEILRSG KSPDLVSFVQ TLCKGLSQPT 180<br>TNLVAGCLQL NPRTFLPEQN QDMPPHLPTA SASFPVHPYS YQSPGLPSPP YGTMDSSHVF 240<br>HVKPPPHAYS AALEPFFESP LTDCTSPSFD GPLSPPLSIN GNFSFKHEPS AEFEKNYAFT 300<br>MHYPAATLAG AQSHGSIFSG TAAPRCEIPI DNIMSFDSHS HHERVMSAQL NAIFHD 356 | |
| **3-3** | **Sequences** | |
| 3-3-1 | Sequence Number [ID] | 3 |
| 3-3-2 | Molecule Type | DNA |
| 3-3-3 | Length | 1071 |
| 3-3-4-1 | Features Location/Qualifiers | **source** 1..1071<br>mol_type= other DNA<br>note= Human NeuroD1 Gene (CDS)<br>organism= Homo sapiens |
| | NonEnglishQualifier Value | |
| 3-3-5 | **Residues**<br>atgaccaaat cgtacagcga gagtgggctg atgggcgagc ctcagcccca aggtcctcca 60<br>agctggacag acgagtgtct cagttctcag gacgaggagc acgaggcaga caagaaggag 120<br>gacgacctcg aaaccatgaa cgcagaggag gactcactga ggaacggggg agaggaggag 180<br>gacgaagatg aggacctgga agaggaggaa gaagaggaag aggaggatga cgatcaaaag 240<br>cccaagagac gcggccccaa aaagaagaag atgactaagg ctcgcctgga gcgttttaaa 300<br>ttgagacgca tgaaggctaa cgcccgggag cggaaccgca tgcacggact gaacgcggcg 360<br>ctagacaacc tgcgcaaggt ggtgccttgc tattctaaga cgcagaagct gtccaaaatc 420<br>gagactctgc gcttggccaa gaactacatc tgggctctgt cggagatcct gcgctcaggc 480<br>aaaagcccag acctggtctc cttcgttcag acgctttgca agggcttatc ccaacccacc 540<br>accaacctgg ttgcgggctg cctgcaactc aatcctcgga cttttctgcc tgagcagaac 600<br>caggacatgc ccccccacct gccgacggcc agcgcttcct tccctgtaca cccctactcc 660<br>taccagtcgc ctgggctgcc cagtccgcct tacggtacca tggacagctc ccatgtcttc 720<br>cacgttaagc ctccgccgca cgcctacagc gcagcgctgg agcccttctt tgaaagccct 780<br>ctgactgatt gcaccagccc ttcctttgat ggacccctca gcccgccgct cagcatcaat 840<br>ggcaacttct ctttcaaaca cgaaccgtcc gccgagtttg agaaaaatta tgcctttacc 900<br>atgcactatc ctgcagcgac actggcaggg gcccaaagcc acggatcaat cttctcaggc 960<br>accgctgccc ctcgctgcga gatccccata gacaatatta tgtccttcga tagccattca 1020<br>catcatgagc gagtcatgag tgcccagctc aatgccatat ttcatgatta a 1071 | |
| **3-4** | **Sequences** | |
| 3-4-1 | Sequence Number [ID] | 4 |
| 3-4-2 | Molecule Type | RNA |
| 3-4-3 | Length | 1310 |

| | | |
|---|---|---|
| 3-4-4-1 | Features Location/Qualifiers | **source** 1..1310<br>mol_type= mRNA<br>note= Human NeuroD1 mRNA sequence<br>organism= Homo sapiens |
| | NonEnglishQualifier Value | |
| 3-4-5 | **Residues**<br>aggactcttc tggtccccac agactcagag agaacccgcc accatgacca aatcgtacag 60<br>cgagagtggg ctgatgggcg agcctcagcc ccaaggtcct ccaagctgga cagacgagtg 120<br>tctcagttct caggacgagg agcacgaggc agacaagaag gaggacgacc tcgaaaccat 180<br>gaacgcagag gaggactcac tgaggaacgg gggagaggag gaggacgaag atgaggacct 240<br>ggaagaggag gaagaagagg aagaggagga tgacgatcaa aagcccaaga gacgcggccc 300<br>caaaaagaag aagatgacta aggctcgcct ggagcgtttt aaattgagac gcatgaaggc 360<br>taacgcccgg gagcggaacc gcatgcacgg actgaacgcg gcgctagaca acctgcgcaa 420<br>ggtggtgcct tgctattcta agacgcagaa gctgtccaaa atcgagactc tgcgcttggc 480<br>caagaactac atctgggctc tgtcggagat cctgcgctca ggcaaaagcc cagacctggt 540<br>ctccttcgtt cagacgcttt gcaagggctt atcccaaccc accaccaacc tggttgcggg 600<br>ctgcctgcaa ctcaatcctc ggacttttct gcctgagcag aaccaggaca tgcccccccca 660<br>cctgccgacg gccagcgctt ccttccctgt acacccctac tcctaccagt cgcctgggct 720<br>gcccagtccg ccttacggta ccatggacag ctcccatgtc ttccacgtta agcctccgcc 780<br>gcacgcctac agcgcagcgc tggagccctt ctttgaaagc cctctgactg attgcaccag 840<br>ccccttcctt gatggacccc tcagcccgcc gctcagcatc aatggcaact tctctttcaa 900<br>acacgaaccg tccgccgagt ttgagaaaaa ttatgccttt accatgcact atcctgcagc 960<br>gacactggca ggggcccaaa gccacggatc aatcttctca ggcaccgctg cccctcgctg 1020<br>cgagatcccc atagacaata ttatgtcctt cgatagccat tcacatcatg agcgagtcat 1080<br>gagtgcccag ctcaatgcca tatttcatga ttaatgatag gctggagcct cggtggccat 1140<br>gcttcttgcc ccttgggcct ccccccagcc cctcctcccc ttcctgcacc cgtacccccg 1200<br>tggtctttga ataaagtctg agtgggcggc aaaaaaaaaa aaaaaaaaaa aaaaaaaaaa 1260<br>aaaaaaaaaa aaaaaaaaaa aaaaaaaaaa aaaaaaaaaa aaaaaaaaaa 1310 | |
| **3-5** | **Sequences** | |
| 3-5-1 | Sequence Number [ID] | 5 |
| 3-5-2 | Molecule Type | RNA |
| 3-5-3 | Length | 1310 |
| 3-5-4-1 | Features Location/Qualifiers | **source** 1..1310<br>mol_type= other RNA<br>note= Human NeuroD1 mRNA containing modified nucleotides<br>organism= synthetic construct |
| | NonEnglishQualifier Value | |
| 3-5-4-2 | Features Location/Qualifiers | **modified_base** 6..1223<br>mod_base= m1f<br>note= all uridine bases are modified into N1-methyl pseudouridine(m1f) |
| | NonEnglishQualifier Value | |
| 3-5-5 | **Residues**<br>aggactcttc tggtccccac agactcagag agaacccgcc accatgacca aatcgtacag 60<br>cgagagtggg ctgatgggcg agcctcagcc ccaaggtcct ccaagctgga cagacgagtg 120<br>tctcagttct caggacgagg agcacgaggc agacaagaag gaggacgacc tcgaaaccat 180<br>gaacgcagag gaggactcac tgaggaacgg gggagaggag gaggacgaag atgaggacct 240<br>ggaagaggag gaagaagagg aagaggagga tgacgatcaa aagcccaaga gacgcggccc 300<br>caaaaagaag aagatgacta aggctcgcct ggagcgtttt aaattgagac gcatgaaggc 360<br>taacgcccgg gagcggaacc gcatgcacgg actgaacgcg gcgctagaca acctgcgcaa 420<br>ggtggtgcct tgctattcta agacgcagaa gctgtccaaa atcgagactc tgcgcttggc 480<br>caagaactac atctgggctc tgtcggagat cctgcgctca ggcaaaagcc cagacctggt 540<br>ctccttcgtt cagacgcttt gcaagggctt atcccaaccc accaccaacc tggttgcggg 600<br>ctgcctgcaa ctcaatcctc ggacttttct gcctgagcag aaccaggaca tgcccccccca 660<br>cctgccgacg gccagcgctt ccttccctgt acacccctac tcctaccagt cgcctgggct 720<br>gcccagtccg ccttacggta ccatggacag ctcccatgtc ttccacgtta agcctccgcc 780<br>gcacgcctac agcgcagcgc tggagccctt ctttgaaagc cctctgactg attgcaccag 840<br>ccccttcctt gatggacccc tcagcccgcc gctcagcatc aatggcaact tctctttcaa 900<br>acacgaaccg tccgccgagt ttgagaaaaa ttatgccttt accatgcact atcctgcagc 960<br>gacactggca ggggcccaaa gccacggatc aatcttctca ggcaccgctg cccctcgctg 1020<br>cgagatcccc atagacaata ttatgtcctt cgatagccat tcacatcatg agcgagtcat 1080<br>gagtgcccag ctcaatgcca tatttcatga ttaatgatag gctggagcct cggtggccat 1140<br>gcttcttgcc ccttgggcct ccccccagcc cctcctcccc ttcctgcacc cgtacccccg 1200<br>tggtctttga ataaagtctg agtgggcggc aaaaaaaaaa aaaaaaaaaa aaaaaaaaaa 1260<br>aaaaaaaaaa aaaaaaaaaa aaaaaaaaaa aaaaaaaaaa aaaaaaaaaa 1310 | |

## Claims

1. A compound of a formula (I) or a salt thereof:

[Chem. 1]

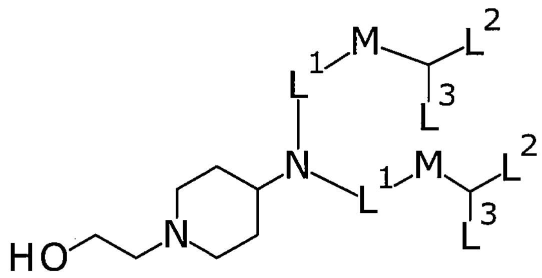

(I)

(in the formula,

$L^1$ is a $C_{5-10}$ alkylene,
$L^2$ and $L^3$ are each independently a $C_{5-15}$ alkyl, and
M is -C(=O)O- or -OC(=O)-).

2. The compound or the salt thereof according to claim 1, wherein $L^1$ in the formula (I) is a $C_{6-9}$ alkylene.

3. The compound or the salt thereof according to claim 2, wherein $L^2$ and $L^3$ in the formula (I) are each independently a $C_{6-10}$ alkyl.

4. The compound or the salt thereof according to claim 3,

wherein the compound is selected from the group consisting of
di(heptadecan-9-yl) 8,8'-{[1-(2-hydroxyethyl)piperidin-4-yl]azanediyl}di(octanoate),
di(tridecan-7-yl) 9,9'- {[1-(2-hydroxyethyl)piperidin-4-yl]azanediyl }di(nonanoate) and
{[1-(2-hydroxyethyl)piperidin-4-yl]azanediyl}di(nonane-9,1-diyl) bis(2-hexyldecanoate).

5. The compound or the salt thereof according to claim 4, wherein the compound is di(heptadecan-9-yl) 8,8'-{[1-(2-hydroxyethyl)piperidin-4-yl]azanediyl}di(octanoate).

6. The compound or the salt thereof according to claim 4, wherein the compound is di(tridecan-7-yl) 9,9'-{[1-(2-hydroxyethyl)piperidin-4-yl]azanediyl}di(nonanoate).

7. The compound or the salt thereof according to claim 4, wherein the compound is {[1-(2-hydroxyethyl)piperidin-4-yl] azanediyl} di(nonane-9, 1-diyl) bis(2-hexyldecanoate).

8. Lipid nanoparticles containing the compound or the salt thereof according to claim 1.

9. Lipid nanoparticles containing the compound or the salt thereof according to claim 1, a neutral lipid and a PEGylated lipid.

10. The lipid nanoparticles according to claim 9 which encapsulate a nucleic acid.

11. The lipid nanoparticles according to claim 10, wherein the nucleic acid is mRNA.

12. The lipid nanoparticles according to claim 11 which can express a protein in an astrocyte.

13. The lipid nanoparticles according to claim 10, wherein the nucleic acid is a nucleic acid which is useful for preventing and/or treating an astrocyte-related disease.

14. Lipid nanoparticles according to claim 11 encapsulating a nucleic acid and containing a compound of or a salt thereof

according to claim 1, neutral lipids and a PEGylated lipid,

wherein the nucleic acid is mRNA encoding NeuroD1 protein,
the compound or the salt thereof according to claim 11 is a compound selected from the group consisting of di(heptadecan-9-yl) 8,8'-{[1-(2-hydroxyethyl)piperidin-4-yl]azanediyl}di(octanoate), di(tridecan-7-yl) 9,9'-{[1-(2-hydroxyethyl)piperidin-4-yl]azanediyl}di(nonanoate) and {[1-(2-hydroxyethyl)piperidin-4-yl]azanediyl}di(nonane-9,1-diyl) bis(2-hexyldecanoate) or a salt thereof,
the neutral lipids are DSPC and cholesterol, and
the PEGylated lipid is DMG-PEG2000.

**15.** Lipid nanoparticles according to claim 14,

wherein the nucleic acid is mRNA encoding NeuroD1 protein, and
the compound of the formula (I) or the salt thereof is di(heptadecan-9-yl) 8,8'-{[1-(2-hydroxyethyl)piperidin-4-yl]azanediyl}di(octanoate) or a salt thereof,

**16.** The lipid nanoparticles according to claim 11 or 15,
wherein the nucleic acid is mRNA encoding NeuroD1 protein containing a base sequence encoding a protein having the amino acid sequence of SEQ ID NO: 2.

**17.** The lipid nanoparticles according to claim 11 which contain 20.0 to 70.0 mol% of the compound or the salt thereof according to claim 1, 27.0 to 79.5 mol% of the neutral lipid and 0.5 to 3.0 mol% of the PEGylated lipid based on the total amount of the lipid nanoparticles.

**18.** The lipid nanoparticles according to claim 11 which contain 35.0 to 70.0 mol% of the compound or the salt thereof according to claim 1, 27.5 to 64.0 mol% of the neutral lipid and 1.0 to 2.5 mol% of the PEGylated lipid based on the total amount of the lipid nanoparticles.

**19.** A pharmaceutical composition containing the lipid nanoparticles according to claim 8, 14 or 15.

**20.** A pharmaceutical composition containing the lipid nanoparticles according to claim 8, 14 or 15 and one or more pharmaceutically acceptable pharmaceutical additives.

**21.** The pharmaceutical composition according to claim 19 or 20 for the prevention and/or the treatment of an astrocyte-related disease.

**22.** Use of the lipid nanoparticles containing the compound of the formula (I) or the salt thereof according to claim 8, 14 or 15 for the manufacture of a pharmaceutical composition for preventing and/or treating an astrocyte-related disease.

**23.** The lipid nanoparticles according to claim 8, 14 or 15 containing the compound of the formula (I) or the salt thereof for use in prevention and/or treatment of an astrocyte-related disease.

**24.** Use of the lipid nanoparticles according to claim 8, 14 or 15 containing the compound of the formula (I) or the salt thereof for the prevention and/or the treatment of an astrocyte-related disease.

**25.** A method for preventing and/or treating an astrocyte-related disease including administering an effective amount of the lipid nanoparticles according to claim 8, 14 or 15 containing the compound of the formula (I) or the salt thereof to a subject.

[Fig. 1]

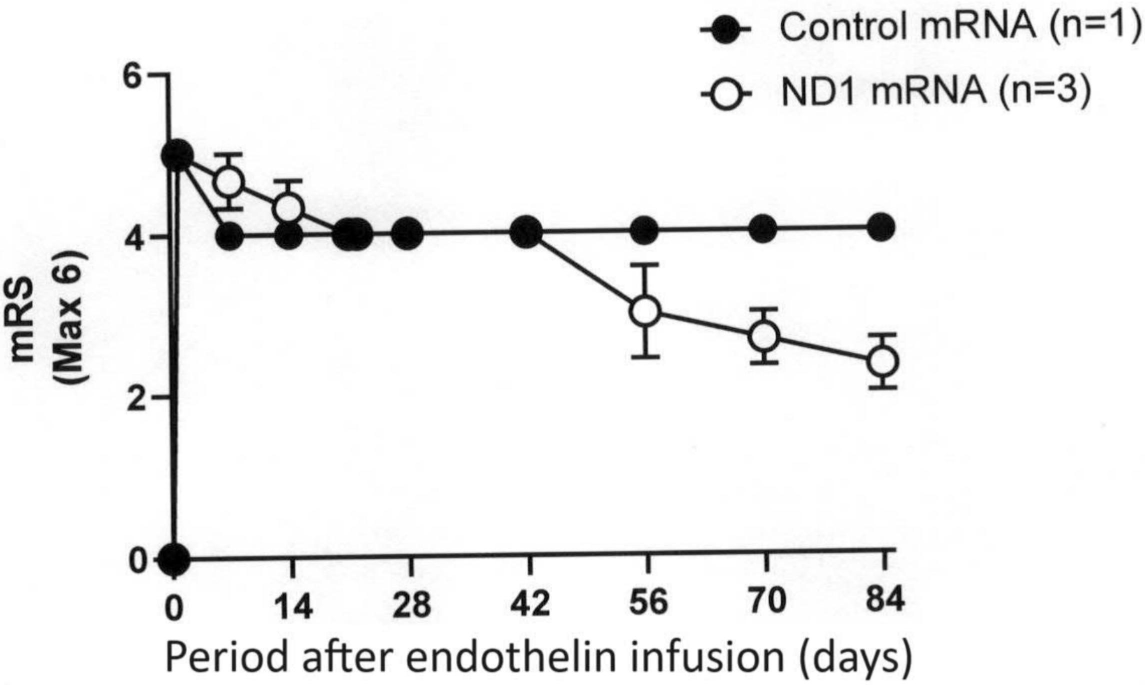
Control mRNA (n=1)
ND1 mRNA (n=3)
mRS
(Max 6)
6
4
2
0
0
14
28
42
56
70
84
Period after endothelin infusion (days)

# INTERNATIONAL SEARCH REPORT

International application No.
**PCT/JP2023/046859**

## A. CLASSIFICATION OF SUBJECT MATTER

***C07D 211/58***(2006.01)i; ***A61K 9/51***(2006.01)i; ***A61K 9/127***(2006.01)i; ***A61K 31/7088***(2006.01)i; ***A61K 47/18***(2017.01)i; ***A61K 47/34***(2017.01)i; ***A61K 47/44***(2017.01)i; ***A61K 48/00***(2006.01)i; ***A61P 25/00***(2006.01)i; ***A61P 25/08***(2006.01)i; ***A61P 25/28***(2006.01)i; ***A61P 43/00***(2006.01)i; ***C12N 15/12***(2006.01)i; ***C12N 15/88***(2006.01)i
FI: C07D211/58 CSP; A61K9/51; A61K31/7088; A61P25/00; A61P43/00 105; A61K47/34; A61K48/00; A61K47/44 ZNA; A61K47/18; A61P25/08; A61P25/28; A61K9/127; C12N15/88 Z; C12N15/12

According to International Patent Classification (IPC) or to both national classification and IPC

## B. FIELDS SEARCHED

Minimum documentation searched (classification system followed by classification symbols)

C07D211/58; A61K9/51; A61K9/127; A61K31/7088; A61K47/18; A61K47/34; A61K47/44; A61K48/00; A61P25/00; A61P25/08; A61P25/28; A61P43/00; C12N15/12; C12N15/88

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

## C. DOCUMENTS CONSIDERED TO BE RELEVANT

| Category* | Citation of document, with indication, where appropriate, of the relevant passages | Relevant to claim No. |
|---|---|---|
| X | WO 2021/204175 A1 (SUZHOU ABOGEN BIOSCIENCES CO., LTD.) 14 October 2021 (2021-10-14) claims, paragraphs [0002]-[0003], [0091]-[00368], examples | 1-11, 17-20 |
| Y | | 12-16, 21-25 |
| X | WO 2021/204179 A1 (SUZHOU ABOGEN BIOSCIENCES CO., LTD.) 14 October 2021 (2021-10-14) paragraphs [0002]-[0003], [0233]-[0474], examples | 1-11, 17-20 |
| Y | | 12-16, 21-25 |
| Y | WO 2021/108609 A1 (THE PENN STATE RESEARCH FOUNDATION) 03 June 2021 (2021-06-03) claims, paragraphs [00195]-[00199], examples | 12-16, 21-25 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 March 2024** | **02 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2023/046859

C. DOCUMENTS CONSIDERED TO BE RELEVANT

| Category* | Citation of document, with indication, where appropriate, of the relevant passages | Relevant to claim No. |
|---|---|---|
| Y | JP 2020-514328 A (THE PENN STATE RESEARCH FOUNDATION) 21 May 2020 (2020-05-21) claims, paragraphs [0106]-[0110], examples | 12-16, 21-25 |
| A | JP 2018-533573 A (ACUITAS THERAPEUTICS, INC.) 15 November 2018 (2018-11-15) entire text, all drawings | 1-25 |

Form PCT/ISA/210 (second sheet) (July 2022)

**INTERNATIONAL SEARCH REPORT**

International application No.
**PCT/JP2023/046859**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2023/046859**

| Patent document cited in search report | Publication date (day/month/year) | Patent family member(s) | Publication date (day/month/year) |
|---|---|---|---|
| WO 2021/204175 A1 | 14 October 2021 | CN 114206827 A | |
| | | US 2022/0153686 A1 | |
| | | KR 10-2022-0166802 A | |
| | | JP 2023-529522 A | |
| WO 2021/204179 A1 | 14 October 2021 | CN 113874507 A | |
| | | US 2022/0218816 A1 | |
| WO 2021/108609 A1 | 03 June 2021 | CN 115135382 A | |
| | | JP 2023-502782 A | |
| JP 2020-514328 A | 21 May 2020 | WO 2018/160712 A1 claims, paragraphs [00193]-[00197], examples | |
| | | CN 110366423 A | |
| | | EP 3589307 B1 | |
| | | US 2020/0054711 A1 | |
| JP 2018-533573 A | 15 November 2018 | US 2017/0119904 A1 | |
| | | WO 2017/075531 A1 | |
| | | CN 108368028 A | |
| | | EP 3368507 B1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021204175 A
- WO 2021204179 A
- WO 2022081750 A
- JP 2022211994 A
- JP 2023041432 A
- WO 2014015261 A

### Non-patent literature cited in the description


- *Frontiers in Cellular Neuroscience*, 2022, vol. 16, 850866
- *Toxicologic Pathology*, 2011, vol. 39 (1), 115-123
- *Neuron*, 2014, vol. 81, 229-248
- *Nature Reviews Molecular Cell Biology*, 2014, vol. 15 (5), 313-326
- *Nucleic Acids Res.*, 2015, vol. 43, pW580-W584
- **P. G. M. WUTS** ; **T. W. GREENE**. Greene's Protective Groups in Organic Synthesis. 2014
- **S. R. SANDLER** ; **W. KARO**. Organic Functional Group Preparations. Academic Press Inc, 1991, vol. 1
- **JIKKEN KAGAKU KOZA**. Courses in Experimental Chemistry. Chemical Society of Japan, 2005, vol. 14
- **S. R. SANDLER** ; **W. KARO**. Organic Functional Group Preparations. Academic Press Inc., 1991, vol. 1
- **JIKKEN KAGAKU KOZA**. Courses in Experimental Chemistry. Chemical Society of Japan, 2005, vol. 16
- **M. HUDLICKY**. Reductions in Organic Chemistry. ACS, 1996
- **R. C. LAROCK**. Comprehensive Organic Transformations. VCH Publishers, Inc, 1999
- **T. J. DONOHOE**. Oxidation and Reduction in Organic Synthesis (Oxford Chemistry Primers 6). 2000
- **LYNETTE C. FOO.** Purification of Rat and Mouse Astrocytes by Immunopanning. Cold Spring Harbor Protocols, 2013, vol. 5, 421-432
- **LYNETTE C. FOO**. Purification of Rat and Mouse Astrocytes by Immunopanning. Cold Spring Harbor Protocols, 2013, vol. 5, 421-432